# EUROPÄISCHE PATENTSCHRIFT

(11) **EP 2 752 208 B1**
(45) Veröffentlichungstag und Bekanntmachung des Hinweises auf die Patenterteilung: **16.09.2015**
(21) Anmeldenummer: 14150491.0
(22) Anmeldetag: 08.01.2014
(51) Int. Cl.: A61M 1/10

(54) **Herzunterstützungsvorrichtung mit einer selbst-expandierenden Schale**
Cardiac assistance device with a self-expanding shell
Dispositif d'assistance au chauffage doté d'une coque auto-expansible

(30) Priorität: 08.01.2013 DE 102013200154
(43) Veröffentlichungstag der Anmeldung: 09.07.2014
(73) Patentinhaber: AdjuCor GmbH, 85748 Garching b. München (DE)
(72) Erfinder: Wildhirt, Stephen, 80997 München (DE); Meister, Markus, 80997 München (DE)
(74) Vertreter: Peterreins, Frank

(56) Entgegenhaltungen:
- US-A- 3 053 249
- US-A- 6 126 590
- US-A1- 2009 036 730
- US-A1- 2010 152 523
- US-A1- 2012 130 485
- US-A1- 2012 283 506
- US-B1- 6 258 021

## Beschreibung

### Zusammenfassung

Die vorliegende Erfindung betrifft eine Vorrichtung zur Unterstützung der Funktion eines Herzens. Die Vorrichtung umfasst eine Schale und eine Hülle mit zumindest einer expandierbaren Einheit. Die expandierbare Einheit kann einen Druck auf eine Fläche ausüben. Die expandierbare Einheit kann eine Augmentationseinheit oder eine Positionierungseinheit sein.

### Hintergrund

Die Erfindung betrifft eine Vorrichtung zur Unterstützung der Funktion eines Herzens. Insbesondere dient die erfindungsgemäße Vorrichtung der Unterstützung einer Pumpfunktion des Herzens.

Krankheitsbedingt kann die Pumpfunktion eines Herzens reduziert sein, was auch als Herzinsuffizienz bezeichnet wird. Die Herzinsuffizienz ist sowohl aus medizinischer als auch aus ökonomischer Sicht von größer und wachsender Bedeutung. In der zweiten Dekade dieses Jahrhunderts werden weltweit 23 Mio. Menschen an der Herzinsuffizienz leiden, die jährliche Neuerkrankungsrate wird dann bei 2 Mio. Menschen liegen. Alleine in den USA sind derzeit ca. 5 Mio. Menschen an Herzinsuffizienz erkrankt. Hier beträgt die jährliche Neuerkrankungsrate ca. 550.000 Menschen. Bereits in diesem Jahrzehnt werden sich alleine in den USA die Zahlen bei den über 50-Jährigen auf über 10 Mio. verdoppeln. Das gleiche gilt für den europäischen Kontinent.

Ursächlich für eine Herzinsuffizienz kann eine verschlechterte Kontraktionsfähigkeit oder Füllung des Herzens durch eine Schädigung des Herzmuskels sein. Ein erhöhter Blutdruck kann zu einem erhöhten Pumpwiderstand führen, der sich ebenfalls negativ auf die Pumpfunktion des Herzens auswirken kann. Die Pumpfunktion eines Herzens kann auch durch undichte Klappen, wie z.B. einer undichten Aortenklappe oder Mitralklappe, vermindert sein. Beeinträchtigungen der Erregungsleitung im Herzen können ebenfalls zu einer verminderten Pumpleistung des Herzens führen. Wird das Herz von außen in seiner Beweglichkeit eingeengt, z.B. durch eine Flüssigkeitsansammlung im Herzbeutel, kann auch dies eine verminderte Pumpfunktion zur Folge haben. Eine Herzinsuffizienz führt häufig zu Luftnot (insbesondere bei einer Linksherzinsuffizienz) oder zu Wasseransammlungen in der Lunge (insbesondere bei einer Linksherzinsuffizienz) oder in den Beinen oder im Bauchraum (insbesondere bei einer Rechtsherzinsuffizienz).

Verschiedene Arten der Herzinsuffizienz können medikamentös oder operativ behandelt werden. Störungen in der Erregungsleitung können unter gewissen Voraussetzungen mit einem Herzschrittmacher behandelt werden. Eine defekte Herzklappe kann operativ durch eine Herzklappenprothese ersetzt werden. Eine verminderte Pumpleistung kann durch Implantation einer Herzpumpe behandelt werden. Ein die verschiedenen Ursachen der Herzinsuffizienz übergreifender Ansatz der Behandlung ist die Unterstützung der Pumpfunktion des Herzens durch ein Implantat, das auf das Herz einen mechanischen Druck ausübt und damit dessen Pumpleistung verbessert.

Bisher bekannte mechanische Herzunterstützungsvorrichtungen sind beispielsweise in den US Patenten US 5,749,839 B1 und US 6,626,821 B1 und in der WO Anmeldung 00/25842 offenbart. Diese Dokumente offenbaren mechanische Herzunterstützungsvorrichtungen, die den Nachteil haben, dass eine Implantation der Vorrichtung eine Operation am offenen Brustkorb erfordert. Die gegenwärtigen Unterstützungssysteme sind komplex und können nur mit einer aufwendigen chirurgischen Operation implantiert werden. Sämtlich werden sie in den Blutkreislauf der Patienten integriert. Verbesserte Zentrifugalpumpen oder magnetisch gelagerte Impellersysteme befördern das Blut kontinuierlich. Der Kontakt des Blutes mit der körperfremden Oberfläche der Systeme stellt eine große technische und medizinische Herausforderung dar. Gängige Komplikationen dieser Systeme sind Schlaganfälle, Blutungen und Infektionen. Sie führen nicht selten zur Langzeithospitalisierung und zu häufigen Wiederaufnahmen bereits aus dem Krankenhaus entlassener Patienten.

Eine aus dem Stand der Technik bekannte Herzunterstützungsvorrichtung ist offenbart in US 2010/0152523. Der Oberbegriff des Anspruchs 1 ist auf der Offenbarung dieses Dokument basiert. Andere bekannte Herzunterstützungsvorrichtungen, wie z.B. die in US 2008/0021266 A1, DE 10 2009 043 795 A1 und US 2011/0021864 A1 offenbarten Vorrichtungen haben den Nachteil, dass keine Ausführungsformen existieren, welche einer Kompromittierung der Vena cava inferior durch die implantierte Vorrichtung vorbeugen. Die Vena cava inferior mündet von dorsal in den rechten Vorhof. Eine Kompromittierung der Vena cava inferior durch die Vorrichtung würde zu einer unteren Einflussstauung führen (Behinderung der Befüllung des rechten Vorhofs).

Ein weiterer Nachteil bekannter Herzunterstützungssysteme ist, dass keine Vorkehrungen gegen eine axiale Dislokation der Vorrichtung gegenüber dem Herzen getroffen werden. Eine Dislokation führt zu einer schlechteren Passform der Vorrichtung zum Herzen und zum Verlust der Unterstützungsleistung.

Aufgabe der vorliegenden Erfindung ist es eine Herzunterstützungsvorrichtung bereitzustellen die nicht die Nachteile der bekannten Herzunterstützungsvorrichtungen aufweist.

Die vorliegende Erfindung betrifft eine Vorrichtung zur Unterstützung der Funktion eines Herzens. Generell kann die Vorrichtung minimal-invasiv mit Hilfe eines Katheters implantiert werden. Die Vorrichtung hat im Allgemeinen keinen direkten Kontakt mit Blutgefäßen und wird nicht in ein Gefäßsystem eingeführt.

### Zusammenfassung der Erfindung

Die Erfindung beruht auf den Merkmalen der unabhängigen Ansprüche 1 und 14. In soweit im nachfolgenden Teil der Beschreibung der Begriff "Erfindung" verwendet und/oder Ausführungsformen als Teil der Erfindung beschrieben werden und/oder Merkmale als lediglich optional genannt werden, müssen diese Passagen unter Berücksichtigung der Ansprüche interpretiert werden, die alleine den Schutzumfang der Erfindung definieren.

Die Vorrichtung zur Unterstützung der Funktion eines Herzens umfasst eine Schale und eine Hülle mit zumindest einer expandierbaren Einheit. Die Schale und die Hülle haben eine im Wesentlichen gleichartige Form. Die Form der Schale und der Hülle entspricht zumindest einem Teil eines Herzens, vorzugsweise dem unteren Teil des Herzens von der Herzspitze bis etwa zur Klappenebene. Die Hülle kann in die Schale eingebracht werden. Die gleichartige Form der Schale und der Hülle ermöglicht ein passgenaues Zusammenfügen der Hülle und der Schale. Dies vermindert Verschiebungen der Hülle bezüglich der Schale und auch des Herzens, die während dem Betrieb der Vorrichtung auftreten können. Die zumindest eine expandierbare Einheit verbleibt daher auch während dem Betrieb an der für sie vorgesehenen Position. Vorzugsweise werden die Schale und die Hülle maßgefertigt, damit sie das Herz eines Patienten bestmöglich umschließen können. Die Schale kann Befestigungsvorrichtungen für die Hülle haben. So kann die Schale am oberen Rand zumindest eine Schlaufe haben. Die Hülle kann an ihrem oberen Rand zumindest eine Tasche oder Schlaufe haben, die in die zumindest eine Schlaufe der Schale eingehängt werden kann.

Die Hülle kann an der Schale durch Umstülpen ihres oberen Randes über den oberen Rand der Schale befestigt werden. Der umgestülpte Teil der Hülle kann mit der Schale fixiert werden, beispielsweise durch verkleben. Der umgestülpte Teil der Hülle kann - wenn die Schale aus einem Gitterwerk besteht und somit Öffnungen hat - auch mit dem nichtumgestülpten Teil der Hülle fixiert werden, beispielsweise durch Verkleben oder Verschweißen. Die Befestigung durch Umstülpen eines Teils der Hülle um den oberen Rand der Schale ermöglicht ein passgenaues Fixieren der Hülle in der Schale. Durch das Umstülpen der Hülle um den oberen Rand der Schale wird auch das Gewebe vor möglichen Beschädigungen durch den oberen Rand der Schale geschützt. Die Hülle kann aus einem flexibleren Material bestehen als die Schale. Das Umstülpen kann den oberen Rand der Schalenrand abpolstern.

Die Schale kann an der unteren Spitze eine Öffnung haben, durch die ein Teil der Hülle durchragen kann, beispielsweise können zwischen 3 mm und 2 cm der Hülle durch die Öffnung durchragen. Damit wird das Gewebe an der Herzspitze vor möglichen Beschädigungen, die durch den unteren Rand der Schale oder von der Schale weiterführende Verlängerungsstreben hervorgerufen werden könnten, verhindert. Die Hülle ist unten geöffnet und ermöglicht daher den Flüssigkeitsaustausch zwischen dem Raum innerhalb der Hülle und dem Raum zwischen Schale und Perikard. So kann beispielsweise Perikardflüssigkeit durch die Öffnung in der Hülle ein- und ausfließen.

Mit Hilfe der zumindest einen expandierbaren Einheit der Hülle kann ein Druck auf das Herz ausgeübt werden. Die expandierbare Einheit kann in Form einer Kammer sein, die inflatiert werden kann. Zwei verschiedene Arten von Kammern können in/an oder auf der Hülle angebracht sein: Augmentationseinheiten und Positionierungseinheiten.

Eine Augmentationseinheit ist eine Einheit, die periodisch expandiert und entspannt werden kann und so einen Druck auf das Herz ausüben kann. Dieser Druck wird vorzugsweise in Bereichen auf den Herzmuskel ausgeübt, unter denen sich eine Herzkammer befindet. Durch Ausüben von Druck auf eine Herzkammer durch eine Augmentationseinheit wird die natürliche Pumpbewegung des Herzens unterstützt oder ersetzt, und das Blut im Herzen wird aus der Kammer in die abführende Arterie gepumpt. Die zumindest eine expandierbare Einheit kann eine Augmentationseinheit sein, die sich vorzugsweise im oberen Bereich der Hülle nahe der Herzklappenebene befindet. Eine Augmentationseinheit ist eine aktiv expandierbare Einheit, die beispielsweise durch Befüllen mit einem Fluid expandiert werden kann.

Eine Positionierungseinheit ist eine Einheit, die expandiert werden kann, um die Vorrichtung am Herzen zu fixieren und die Passform der Vorrichtung zu gewährleisten. Mit Hilfe einer Positionierungseinheit kann auf Veränderungen des Herzmuskels, wie z.B. der Schrumpfung des Herzmuskels aufgrund von Flüssigkeitsmangel oder der Ausdehnung des Herzmuskels aufgrund von Flüssigkeitsaufnahme des Patienten, reagiert werden. Baut sich der Herzmuskel im Laufe eines Zeitraumes ab oder auf, kann eine Positionierungseinheit weiter expandiert oder entspannt werden, um eine optimale Passform zu gewährleisten. Die Positionierungseinheit kann auch dazu dienen, dass die Vorrichtung über die Dauer eines Herzschlages nicht den Kontakt mit der Herzwand verliert. Die zumindest eine expandierbare Einheit kann eine Positionierungseinheit sein, die sich vorzugsweise im oberen Bereich der Hülle nahe der Herzklappenebene befindet. Die Positionierungseinheit kann eine aktiv expandierbare Einheit sein, die insbesondere durch Befüllen mit einem Fluid expandiert werden kann.

Die Hülle kann auch zumindest einen Sensor umfassen. Dieser Sensor kann geeignet sein, die Herzfrequenz, die Sauerstoffsättigung, das EKG oder den Druck von einer expandierbaren Einheit gegen das Herz ausgeübten Druck, zu messen. Der zumindest eine Sensor kann auch als Elektrode verwendet werden. So kann mit der zumindest einen Elektrode das Aktionspotential am Herzmuskel während des Erregungsprozesses gemessen werden. Ebenso kann mit der zumindest einen Elektrode der Herzmuskel mit Hilfe von elektrischen Impulsen stimuliert werden. Dazu kann die Vorrichtung zur Unterstützung der Funktion eines Herzens eine Steuereinheit umfassen. Die Steuereinheit ermöglicht durch Verwendung der Daten des EKG Sensors ein gezieltes Ansteuern der zumindest einen expandierbaren Einheit.

Die Hülle kann mit einem Gleitmittel zur Verminderung von Reibung beschichtet sein, insbesondere kann die Beschichtung eine Beschichtung mit Graphit oder eine reibungsvermindernde Silikonbeschichtung sein. Ebenso kann eine Hülle oder die Schale mit einem pharmazeutischen Wirkstoff beschichtet sein.

Ein Aspekt der Erfindung betrifft eine Aussparung am oberen Rand der Schale, die verhindert, dass die Schale im implantierten Zustand die Vena cava inferior kompromittiert. Die Aussparung kann eine Länge entlang des oberen Schalenrandes von 2 cm, 3 cm, 4 cm, 5 cm, 6 cm, 7 cm, oder mehr haben. wobei die Aussparung eine Tiefe (vom gedachten nicht-unterbrochenen oberen Schalenrand zu dem Punkt an der Aussparung, welcher der Herzspitze am nächsten liegt) zwischen 1 mm und 40 mm hat, insbesondere zwischen 3mm und 15mm. In Umfangsrichtung ist die Aussparung dort positioniert, wo die Vena cava inferior in den rechten Vorhof mündet. Die Aussparung kann bogenförmig, halbkreisförmig, rechteckig oder polygonal sein.

Die Hülle weist ebenso eine Aussparung auf, die in ihrer Form, Lage und Dimension im Wesentlichen mit der Aussparung in der Schale übereinstimmt, welche den anatomischen Bereich um die Vena cava inferior freistellt. Die Aussparungen in Hülle und Schale können miteinander in Deckung gebracht werden.

Ein anderer Aspekt der Erfindung betrifft zumindest eine Verlängerungsstrebe der Schale. Die zumindest eine Verlängerungsstrebe ragt von der unteren Spitze der Schale in Richtung der Herzlängsachse vom Herzen weg. Die zumindest eine Verlängerungsstrebe Teil der Schale ist, insbesondere die Verlängerung einer Strebe des Gitterwerkes, wobei die zumindest eine Verlängerungsstrebe an die Schale gekoppelt werden kann. Das Material der Verlängerungsstrebe kann aus dem gleichen Material wie die Schale gefertigt sein. Alternativ kann auch ein anderes Material verwendet werden. Die Schale kann zwei, drei, vier, fünf, sechs oder mehr Verlängerungsstreben umfassen. Die zumindest eine Verlängerungsstrebe kann zwischen 1 cm und 10 cm, insbesondere zwischen 4 cm und 7 cm lang sein.

Ausführungsformen der Erfindung bestehen aus einer Schale mit der oben beschriebenen Aussparung und zumindest einer Verlängerungsstrebe. Andere Ausführungsformen haben nur eine Aussparung und keine Verlängerungsstreben. Weitere Ausführungsformen umfassen eine Schale ohne Aussparung und haben zumindest eine Verlängerungsstrebe.

Die Vorrichtung zur Unterstützung der Funktion eines Herzen kann vorzugsweise minimal-invasiv implantiert werden.

Weitere Merkmale und Vorteile der Erfindung ergeben sich aus der nachfolgenden Beschreibung und aus den nachfolgenden Zeichnungen, auf die Bezug genommen wird.

### Beschreibung der Zeichnungen

**Figur 1** zeigt einen menschlichen Torso mit der erfindungsgemäßen Vorrichtung, wobei eine Versorgungseinheit extra-korporal liegt.
**Figur 2** zeigt einen menschlichen Torso mit der erfindungsgemäßen Vorrichtung und einer teilweise implantierten Versorgungseinheit.
**Figur 3** zeigt ein menschliches Herz mit der erfindungsgemäßen Vorrichtung.
**Figur 4a und 4b** zeigt einen Schnitt durch das Herz mit der erfindungsgemäßen Vorrichtung entlang der in Figur 3 dargestellten Linie A-A.
**Figur 5** zeigt einen Schritt der Implantation der erfindungsgemäßen Vorrichtung.
**Figur 6** zeigt einen Schritt der Implantation bei dem ein Perikardverschluss noch nicht verschraubt ist.
**Figur 7** zeigt einen Schritt der Implantation bei dem ein Perikardverschluss verschraubt ist.
**Figur 8** zeigt eine expandierte Schale mit einer Hülle.
**Figur 9** zeigt eine teilweise expandierte Schale mit einer Hülle während der Implantation.
**Figuren 10a****-c** zeigen verschiedene Ansichten eines verschlossenen Perikardverschlusses.
**Figur 11** zeigt ein Werkzeug zum Verschließen eines Perikardverschlusses.
**Figur 12** zeigt ein Steckersystem der erfindungsgemäßen Vorrichtung.
**Figur 13a** zeigt ein Herz mit anatomischen Orientierungspunkten.
**Figur 13b** zeigt einen Schnitt durch das Herz aus Figur 13a.
**Figur 14a** zeigt eine 3D-Ansicht eines Teils eines Herzens mit einem Koordinatensystem.
**Figur 14b** zeigt eine 2D-Abrollung der 3D-Ansicht aus Figur 14a mit einem Koordinatensystem.
**Figur 15a** zeigt eine 3D-Ansicht einer Hülle mit Augmentations- und Positionierungseinheiten.
**Figur 15b** zeigt eine 2D-Abrollung der Hülle mit Augmentations- und Positionierungseinheiten aus Figur 15a.
**Figuren 16a****-b** zeigen eine komprimierte und eine expandierte Augmentationseinheit in Form einer Kammer mit einem balgförmigen Abschnitt.
**Figur 17a** zeigt eine 3D-Ansicht einer Hülle mit Sensoren und/oder Elektroden.
**Figur 17b** zeigt eine 2D-Abrollung der Hülle mit Sensoren und/oder Elektroden aus Figur 17a.
**Figur 18** zeigt ein Ausführungsbeispiel für eine Hülle mit Augmentations- und Positionierungseinheiten.
**Figur 19** zeigt ein Ausführungsbeispiel für eine Hülle mit Sensoren und Elektroden.

### Detaillierte Beschreibung

Die erfindungsgemäße Vorrichtung umfasst mehrere Bauteile, die in den folgenden Abschnitten näher erläutert werden, wobei Ausführungsformen der einzelnen Bauteile miteinander kombiniert werden können.

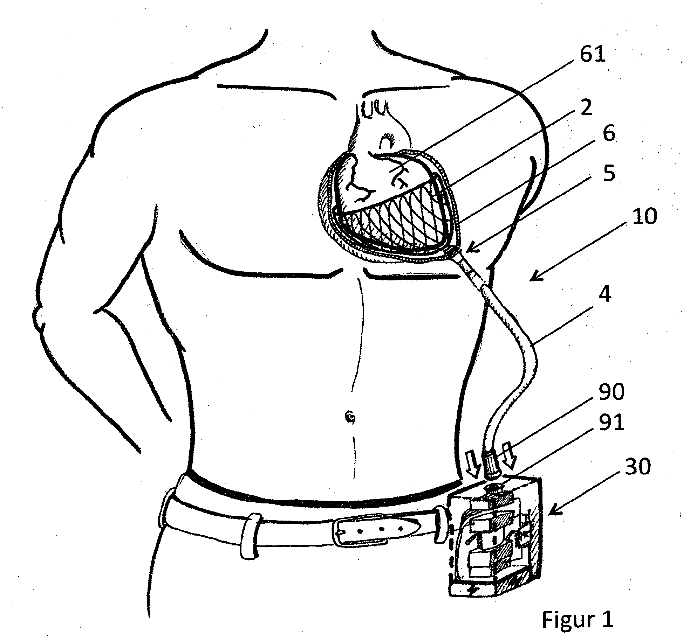

**Figur 1** zeigt eine Ausführungsform (10) der erfindungsgemäßen Vorrichtung im implantierten Zustand. Exemplarisch ist die erfindungsgemäße Vorrichtung in einen menschlichen Körper implantiert. Die erfindungsgemäße Vorrichtung kann jedoch auch in einen tierischen Körper implantiert werden, insbesondere in den Körper eines Säugetieres, wie zum Beispiel einem Hund, einer Katze, einem Nagetier, einem Primaten, einem Paarhufer oder einem Unpaarhufer. Je nach Spezies können dann spezielle Anpassungen der Form und Funktionsweise der erfindungsgemäßen Vorrichtung notwendig sein, um anatomischen und/oder physiologischen Bedürfnisse der einzelnen Spezies gerecht zu werden.

Figur 1 zeigt einen menschlichen Torso mit der erfindungsgemäßen Vorrichtung. Die Vorrichtung besteht aus einer Schale (2), die das Herz (61) zumindest zum Teil umschließen kann, wobei in die Schale (2) Komponenten eingebracht sind, die die Funktion des Herzens (61) unterstützen. Ferner umfasst die Vorrichtung eine Versorgungseinheit (30).

Die Schale (2), die das Herz (61) zumindest zum Teil umschließen kann, kann von einem nicht-expandierten Zustand in einen expandierten Zustand übergehen. Vorzugsweise ist die Schale (2) selbst-expandierend und kann im nicht-expandierten Zustand in ein Zuführsystem eingebracht werden. Die Schale (2) kann aus einem Gitterwerk oder aus einem Geflecht bestehen. Das Geflecht kann als Drahtgeflecht ausgeführt sein. Das Gitterwerk oder das Drahtgeflecht kann zumindest zum Teil aus einer Formgedächtnislegierung gefertigt sein kann. Die Schale (2) umschließt das Herz (61) im implantierten Zustand zumindest teilweise und befindet sich innerhalb des Perikards (6). Ausführungsformen bei denen die Schale (2) außerhalb des Perikards (6) platziert wird sind auch möglich. Diese Ausführungsformen werden nicht gesondert beschrieben, vielmehr gilt die Beschreibung für Ausführungsformen zur Implantation innerhalb und außerhalb des Perikards (6) (mit Ausnahme des nicht notwendigen Perikardverschlusses (5)) bei Ausführungsformen der Schale (2) zur Implantation außerhalb des Perikards (6). Der Aufbau der Schale (2) wird in einem späteren Abschnitt der Beschreibung näher erläutert.

In der expandierbaren Schale (2) befindet sich zumindest eine expandierbare Einheit, mit Hilfe derer ein Druck auf das Herz (61) ausgeübt werden kann. Die expandierbare Einheit kann eine mechanische Einheit sein, die eine expandierte und eine nicht-expandierte Konfiguration annehmen kann. Eine derartige mechanische Einheit kann aus Federelementen, die gespannt und entspannt werden können, oder aus Hebelelementen, die gefaltet und entfaltet werden können, bestehen. Vorzugsweise sind die expandierbaren Einheiten Kammern, die mit einem Fluid gefüllt werden können. Als Fluide zum Befüllen einer Kammer kommen Flüssigkeiten, Gase, oder Festkörper (wie z.B. Nanopartikelgemische), oder Gemische von Flüssigkeiten und/oder Gasen und/oder Festkörpern in Frage. Die zumindest eine expandierbare Einheit kann in der Schale (2) befestigt werden. Vorzugsweise wird die zumindest eine expandierbare Einheit auf einer Hülle befestigt, die in die Schale (2) eingebracht werden kann. Die zumindest eine expandierbare Einheit wird in einem späteren Abschnitt der Beschreibung näher erläutert. Die Hülle, welche in die Schale (2) eingebracht werden kann, wird in einem späteren Abschnitt der Beschreibung näher erläutert.

Die Schale (2) kann ferner zumindest einen Sensor und/oder eine Elektrode umfassen, mit Hilfe dessen zumindest ein Parameter des Herzens (61) erfasst werden kann. Der zumindest eine Sensor kann geeignet sein, die Herzfrequenz, den Herzkammerdruck, die Kontaktkraft zwischen der Herzwand und der zumindest einen expandierbaren Einheit, den systolischen Blutdruck oder den diastolischen Blutdruck zu bestimmen. Der Sensor kann auch geeignet sein, den von einer expandierbaren Einheit auf eine Fläche ausgeübten Druck, den pH-Wert, die Sauerstoffsättigung, den elektrischen Widerstand, die Osmolarität einer Lösung, oder den Durchfluss durch ein Gefäß zu messen. Der zumindest eine Sensor kann in, an oder auf der Schale (2) angebracht sein. Vorzugsweise wird der zumindest eine Sensor auf einer Hülle befestigt, die in die Schale (2) eingebracht werden kann. Der zumindest eine Sensor kann auch eine Elektrode sein, die geeignet ist einen Parameter wie z.B. das Aktionspotential am Herzmuskel während des Erregungsprozesses zu messen oder ein Gewebe mit Strömen zu stimulieren. Der zumindest eine Sensor und/oder die zumindest eine Elektrode wird in einem späteren Abschnitt der Beschreibung näher erläutert.

Figur 1 zeigt eine Versorgungseinheit (30) die außerhalb des Körpers getragen werden kann. Die Versorgungseinheit kann auch teilweise oder vollständig in den Körper implantiert werden, was in nachfolgenden Absätzen noch näher erläutert wird. Wird die Versorgungseinheit (30) außerhalb des Körpers getragen, kann diese an einem Brustgurt, an einem Hüftgurt oder an einem Bauchgurt befestigt werden. Die Versorgungseinheit (30) hat einen Energiespeicher, mit Hilfe dessen die zumindest eine expandierbare Einheit angetrieben werden kann. Der Energiespeicher kann in Form eines Akkumulators vorliegen, der elektrische Energie bereitstellt, um die expandierbare Einheit expandieren zu können. Der Akkumulator kann gewechselt werden. Die Versorgungseinheit (30) kann auch einen Druckspeicher enthalten, der ein komprimiertes Gas bereitstellt, um eine inflatierbare Kammer inflatieren zu können. Geeignete Gase sind unter anderem Druckluft, CO₂, oder Edelgase. Das Gehäuse der Versorgungseinheit (30) selbst kann als Druckspeichergehäuse dienen. Die Versorgungseinheit (30) kann weiterhin Pumpen, Ventile, Sensoren und Displays enthalten. Die Versorgungseinheit (30) kann ferner einen Mikroprozessor beinhalten, der geeignet ist, Daten von dem zumindest einen Sensor zu empfangen und zu verarbeiten. Wird die Versorgungseinheit (30) außerhalb des Körpers getragen, kann die zu bereitstellende Energie durch eine direkte Verbindung über ein Kabel (4) transferiert werden, oder verbindungslos durch z.B. elektromagnetische Induktion. Die Daten von dem zumindest einen Sensor können ebenfalls direkt über ein Kabel (4) öder verbindungslos über eine Funktechnik, wie z.B. bluetooth, übertragen werden.

Die erfindungsgemäße Vorrichtung kann ferner ein Kabel (4) umfassen, das die zumindest eine expandierbare Einheit und/oder den zumindest einen Sensor oder die zumindest eine Elektrode mit der Versorgungseinheit (30) verbindet. Ist die Versorgungseinheit (30) direkt mit der zumindest einen expandierbaren Einheit und/oder dem zumindest einen Sensor oder der zumindest einen Elektrode verbunden, kann auf ein Kabel (4) verzichtet werden. Ist die zumindest eine expandierbare Einheit eine mechanische Einheit, die mit Hilfe von elektrischer Energie von einem nicht-expandierten Zustand in einen expandierten Zustand oder von einem expandierten Zustand in einen nicht-expandierten Zustand übergehen kann, so beinhaltet das Kabel (4) Leitungen, die geeignet sind die notwendige Energie von der Versorgungseinheit (30) an die expandierbare Einheit zu transferieren. Ist die zumindest eine expandierbare Einheit eine Kammer, die mittels eines Fluids befüllt werden kann, so beinhaltet das Kabel (4) zumindest eine Leitung, die den Fluss von Fluid aus der Versorgungseinheit (30) in die Kammer ermöglicht. Vorzugsweise beinhaltet das Kabel (4) in diesem Fall zumindest eine pneumatische oder eine hydraulische Leitung. Beinhaltet die Vorrichtung einen Sensor oder eine Elektrode an, in oder auf der Schale, so kann die zumindest eine Leitung, die zu dem Sensor oder der Elektrode führt, ebenfalls in dem Kabel (4) sein. Ausführungsformen können auch getrennte Kabel für die Bereitstellung von Energie für die zumindest eine expandierbare Einheit und für den zumindest einen Sensoren oder die zumindest eine Elektrode haben.

Das Kabel (4), das die Versorgungseinheit (30) mit der zumindest einen expandierbaren Einheit und/oder dem Sensor oder der Elektrode verbindet, kann ein einzelnes durchgängiges Kabel sein oder ein mehrteiliges Kabel sein. Im Falle eines durchgängigen Kabels kann sich ein Kabel (4) an der zumindest einen expandierbaren Einheit und/oder dem zumindest einen Sensor oder einen Elektrode befinden. Am Ende des Kabels (4) kann dann ein Steckerteil (90) angebracht sein, das über den koppelbaren Anschluss (91) an die Versorgungseinheit (30) verbunden werden kann. Alternativ befindet sich nur an der Versorgungseinheit (30) ein Kabel mit einem Steckerteil. In diesem Fall befindet sich der damit koppelbare Anschluss an der Schale (2), der zumindest einen expandierbaren Einheit und/oder dem zumindest einen Sensor oder Elektrode. Im Falle eines mehrteiligen Kabels kann ein Kabel (4) mit Steckerteil (91) an der zumindest einen expandierbaren Einheit und/oder an dem zumindest einem Sensor oder einen Elektrode angebracht werden und auch ein Kabel an der Versorgungseinheit (30) angebracht werden, an dessen Ende sich vorzugsweise ebenfalls ein Steckerteil befindet. Das Kabel (4) wird in einem späteren Abschnitt der Beschreibung näher erläutert. Das Steckerteil (90) wird in einem späteren Abschnitt der Beschreibung näher erläutert.

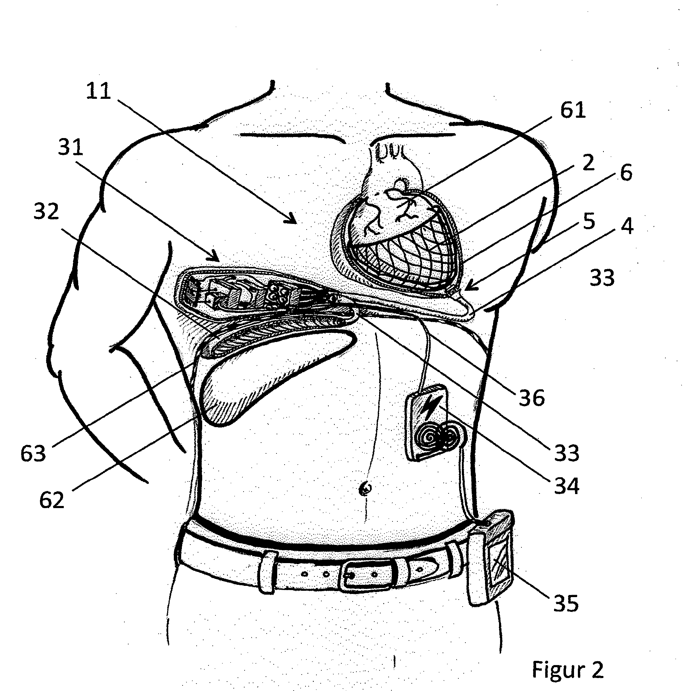

**Figur 2** zeigt eine Ausführungsform (11) der erfindungsgemäßen Vorrichtung im implantierten Zustand, bei der die Versorgungseinheit (31) in den Körper implantiert ist. Bevorzugte Stellen für die Implantation der Versorgungseinheit (31) sind Brusthöhle und Bauchhöhle, die durch das Zwerchfell (63) voneinander getrennt sind.

Die in Figur 2 dargestellte Schale (2), der Perikardverschluss (5), und das Kabel (4) der Vorrichtung stimmen im Wesentlichen mit den in Figur 1 gezeigten Komponenten überein. Die Versorgungseinheit (31) kann einen Energiespeicher beinhalten, mit Hilfe dessen die zumindest eine expandierbare Einheit angetrieben werden kann, die sich in der Schale (2) befindet. Der Energiespeicher kann in Form eines Akkumulators vorliegen, der elektrische Energie bereitstellt, um die expandierbare Einheit expandieren zu können. Die Versorgungseinheit (31) kann ferner Sensoren und einen oder mehrere Mikroprozessor enthalten. Besteht die zumindest eine expandierbare Einheit aus zumindest einer Kammer die mit einem Fluid gefüllt werden kann, so kann die Versorgungseinheit (31) Pumpen, Ventile, und einen Druckspeicher enthalten. Der Druckspeicher kann ein komprimiertes Gas bereitstellen, um eine inflatierbare Kammer inflatieren zu können. Geeignete Gase sind unter anderem Druckluft, CO₂, oder Edelgase. Das Gehäuse der Versorgungseinheit (31) selbst, kann dabei das Druckspeichergehäuse darstellen. Eine bevorzugte Stelle für die Implantation der Versorgungseinheit (31) ist rechts-lateral in der Brusthöhle oberhalb der Leber (62) und oberhalb des Zwerchfells (63). Alternativ oder zusätzlich zum Druckspeicher in der Versorgungseinheit (31) kann ein Druckspeicher (32) bevorzugt rechts-lateral in der Bauchhöhle unterhalb des Zwerchfells (63) und über der Leber (62) implantiert werden. Der Druckspeicher (32) kann mit einem Schlauch (33), der das Zwerchfell (63) durchdringt, mit der Versorgungseinheit (31) verbunden werden. Die zur Durchführung des Schlauches (33) notwendige Öffnung im Zwerchfell kann mit einem Verschluss abgedichtet werden. Dieser Verschluss kann ähnlich dem in dieser Anmeldung beschriebenen Perikardverschluss ausgeführt sein. Die Versorgungseinheit kann mit einem Kabel (4) direkt mit der zumindest einen expandierbaren Einheit und/oder dem Sensor oder der Elektrode verbunden sein. Alternativ kann sich an einem Ende des Kabels (4) ein Steckerteil befinden, das über einen koppelbaren Anschluss an die Versorgungseinheit (31) oder an die zumindest eine expandierbare Einheit und/oder den Sensor oder Elektrode verbunden werden kann. Das Kabel (4) verläuft bevorzugt in der Brusthöhle oberhalb des Zwerchfells (63). Im Falle eines mehrteiligen Kabels, kann ein Kabel mit Steckerteil an der zumindest einen expandierbaren Einheit und/oder dem zumindest einem Sensor oder einen Elektrode angebracht werden und auch ein Kabel mit koppelbarem Steckerteil an der Versorgungseinheit (31) angebracht werden.

Alternativ oder zusätzlich zu einem in der Versorgungseinheit (31) enthaltenen Akkumulator kann ein Akkumulator (34) bevorzugt subkutan in der Bauchdecke implantiert werden. Die in der Versorgungseinheit (31) benötigte Energie kann beispielsweise durch elektromagnetische Induktion von einem extrakorporalen Steuergerät (35) transkutan zum Akkumulator (34) transferiert werden und mittels eines elektrischen Kabels (36) vom Akkumulator (34) zur Versorgungseinheit (31) übertragen werden. Das extrakorporale Steuergerät (35) kann unter anderem einen wechselbaren Akkumulator und/oder ein Ladegerät enthalten. In dem extrakorporalen Steuergerät (34) können unter anderem Mikroprozessoren und Displays enthalten sein, die der Systemüberwachung der Vorrichtung und der Anzeige des Betriebsstatus dienen können. Die Daten von dem zumindest einen Sensor können verbindungslos über eine Funktechnik, wie z.B. bluetooth, zu und zwischen der Versorgungseinheit (31) und dem Steuergerät (34) übertragen werden.

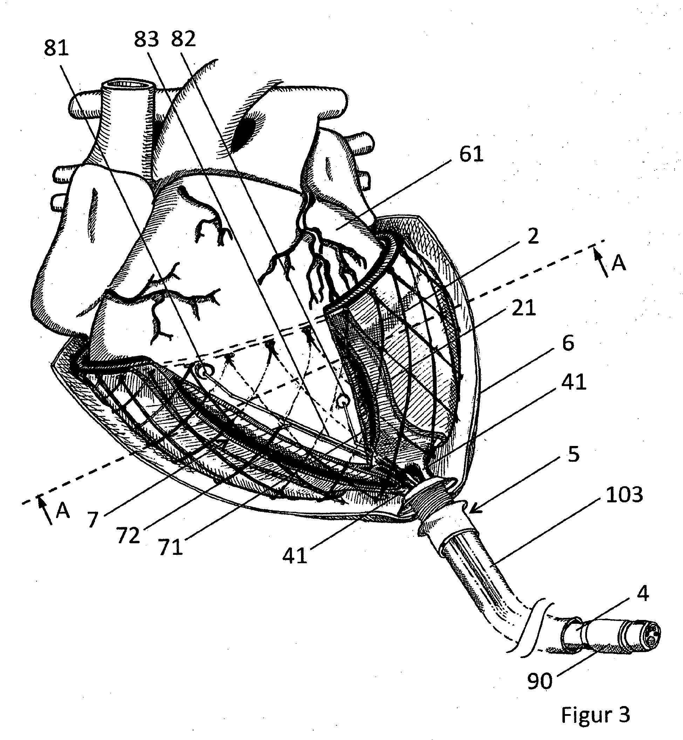

**Figur 3** zeigt exemplarisch ein menschliches Herz (61) sowie eine Schale (2), eine Hülle (7) mit expandierbaren Einheiten (71, 72), Sensoren (81) und/oder Elektroden (82), ein Kabel (4) mit einem Steckerteil (90), einen Katheter (103) eines Zuführsystems und einen Perikardverschluss (5) der erfindungsgemäßen Vorrichtung.

Die Schale (2) ist in dieser Ausführungsform in Form eines Gitterwerks dargestellt. Das Gitterwerk kann aus einem durchgängigen Material bestehen aus dem Teile entfernt wurden oder in welches die Öffnungen hineingeformt wurden. Beispielsweise kann die Schale (2) aus einem Rohr oder einem individuell geformten Schalenmantel gefertigt werden, in welche die Öffnungen geschnitten werden. Bevorzugt wird dabei ein Verfahren in dem kleine Schlitze, beispielsweise mittels Laserschneiden, in ein Rohr geschnitten werden ("slotted tube"). Das geschlitzte Rohr wird dann über formgebende Dorne aufgespannt und mittels einer Wärmebehandlung normalgeglüht, so dass die aufgespannte Form die neue natürliche Konfiguration des Rohres darstellt. Diese Aufspann- und Wärmebehandlungsvorgänge können so oft mit immer größer werdenden Dornen wiederholt werden, bis zuletzt das geschlitzte Rohr über eine Form gespannt werden kann, die einem Abbild des Herzens entspricht und womit in der Wärmebehandlung die Herzform auf das geschlitzte Rohr übertragen wird. Das geschlitzte Rohr wird somit in eine aus einem Gitterwerk bestehende Schale (2) überführt, deren Form einem Abbild des Herzens entspricht. Die Schlitze im Rohr gehen dabei über in die Öffnungen des Gitterwerks. Die Öffnungen werden dabei durch Streben begrenzt. Mehrere Streben einer Öffnung bilden dabei einzelne Zellen des Gitterwerks. Das Gitterwerk kann dabei bevorzugt rautenförmige Zellen aufweisen. Die Form der Zellen kann durch geeignete Schnittführung beim Schlitzen des Rohres modifiziert werden. Alternativ können so Zellen mit einer hexagonalen Wabenstruktur oder Zellen einer polygonalen Struktur erzeugt werden.

Durch geeignete Schnittführung können längere oder kürzere Zellen hergestellt werden. Durch die Länge und Anzahl der Schlitze wird auch die Anzahl der Zellreihen entlang der Herzlängsachse der Schale (2) definiert. Längere Schnitte führen zu längeren Streben, kürzere Schnitte zu kürzeren Streben in der Schale (2). Längere Streben sind dabei biegsamer als kurze Streben. Die Länge der Zellen beträgt 5 mm bis 50 mm, insbesondere 10 mm bis 30 mm. Die Länge der Zellen kann von Zellreihe zu Zellreihe variieren und auch innerhalb einer Zellreihe gleich sein oder variieren.

Über die Anzahl der Schlitze entlang des Umfangs des Rohres wird die Anzahl der Zellen entlang des Umfangs der Schale (2) bestimmt. Weniger Schlitze entlang des Umfangs führen zu einer größeren Breite der resultierenden Streben, eine Erhöhung der Schlitzanzahl entlang des Umfangs führt zu dünneren Streben. Breitere Streben sind dabei weniger flexibel als dünnere Streben. Es können 14 bis 80 Schnitte in einer Reihe entlang des Schalenumfangs angefertigt werden, bevorzugt 20 bis 45 Schnitte entlang des Umfangs, insbesondere 26 bis 36 Schnitte entlang des Umfangs. Bei der Herstellung können die Schnitte gleichmäßig über den Rohrumfang (äquidistant) verteilt werden, womit eine homogene Struktursteifigkeit im Gitterwerk erreicht wird. Alternativ können die Schnitte ungleichmäßig (nicht äquidistant) zueinander verteilt werden, so dass Bereiche mit Zellen mit größerer Strebenbreite (höhere Struktursteifigkeit) und Bereiche mit Zellen geringerer Strebenbreite (niedrigere Struktursteifigkeit) erzeugt werden können. Bereiche in denen eine höhere Struktursteifigkeit erwünscht ist, können beispielweise Bereiche sein die den linken Ventrikel des Herzens abdecken oder Bereiche die das Widerlager für die expandierbaren Einheiten (71, 72) bilden. Bereiche in denen eine geringere Struktursteifigkeit erwünscht ist, sind beispielweise Bereiche am rechten Ventrikel.

Die Wandstärke des Rohres in welches die Schlitze geschnitten werden resultiert in die Strebenhöhe der Schale (2). Eine größere Strebenhöhe führt zu einem steiferen Verhalten des Gitterwerks. Eine geringere Strebenhöhe führt zu einem flexibleren Verhalten des Gitterwerks. Durch die Verwendung eines Rohres mit regional unterschiedlicher Wandstärke können somit Bereiche geringerer oder höherer Struktursteifigkeit erzeugt werden. Die Wandstärke des Rohres kann zwischen 0,2 mm und 2 mm betragen, bevorzugt zwischen 0,4 mm und 1,5 mm, insbesondere zwischen 0,6 mm und 1 mm. Die Wandstärke der Schale ist gleich der Wandstärke des Rohres.

Das Gitterwerk kann aus einem Rohr geschnitten werden, dessen Durchmesser zwischen 4 mm und 20 mm beträgt, insbesondere zwischen 8 mm und 15 mm. Bei gleicher Schlitzanzahl resultiert ein größerer Rohrdurchmesser in einer größeren Strebenbreite. Das Herstellungsverfahren garantiert, dass das Gitterwerk der expandierten Schale (2) auch wieder auf die Größe des Rohrs komprimiert werden kann aus dem es geschnitten wurde. Dies kann insbesondere dann hilfreich sein, wenn die Schale (2) in ein Zuführsystem mit einem Katheter (103) kleineren Durchmessers eingebracht werden soll. Die Schale kann aber auch auf jede Zwischengröße zwischen dem vollständig expandierten, herzförmigen Gitterwerk und dem ursprünglichen Rohrdurchmesser komprimiert werden. Durch die Verwendung z.B. einer Formgedächtnislegierung kann die Schale (2) beim Implantieren/beim Ausbringen aus dem Zuführsystem von selbst in die Herzform expandieren. Das Aufbringen von externen Kräften durch weitere Vorrichtungen ist bei der Verwendung einer Formgedächtnislegierung nicht erforderlich. Insbesondere die Länge der Zellen und/oder Streben des Gitterwerks bestimmt dabei den Öffnungswinkel bei der Expansion der Schale (2), insbesondere bei der selbst-expandierbaren Schale.

Die in Figur 3 dargestellte Schale (2) kann anstelle eines Gitterwerkes auch aus einem Geflecht von Drähten bestehen. Die Drähte formen Kreuzungspunkte, die miteinander fest verbunden werden können. Beispielsweise können die Drähte an den Kreuzungspunkten miteinander verschweißt werden. Das Verbinden der Drähte an Kreuzungspunkten erhöht die Stabilität der Schale (2). Die Kreuzungspunkte können auch nicht miteinander verbunden werden. Dies kann die Flexibilität der Schale (2) erhöhen und damit zu einer leichteren Komprimierbarkeit der Schale (2) führen. Dies kann insbesondere dann hilfreich sein, wenn die Schale (2) in ein Zuführsystem mit einem Katheter (103) kleineren Durchmessers eingebracht werden soll. Die Schale (2) kann auch an einigen der Kreuzungspunkte fest miteinander verbunden sein und an anderen Kreuzungspunkten nicht fest miteinander verbunden sein. Durch eine geeignete Wahl von Kreuzungspunkten, die fest miteinander verbunden werden und Kreuzungspunkten, die nicht fest miteinander verbunden werden, kann die Stabilität und Flexibilität der Schale (2) angepasst werden. Bereiche, die im implantierten Zustand eine erhöhte Stabilität erfordern, können durch das Verbinden der Drähte an Kreuzungspunkten stabilisiert werden. Dies können beispielsweise Bereiche sein, die als Widerlager für expandierbare Einheiten (71, 72) dienen. Solche Widerlager können sich direkt unter einer expandierbaren Einheit (71, 72) befinden oder neben Bereichen mit expandierbaren Einheiten (71, 72). Bereiche, die eine erhöhte Flexibilität erfordern, können Bereiche sein, die beim Einbringen in ein Zuführsystem stärker komprimiert werden müssen als andere Bereiche. Bereiche, die eine erhöhte Flexibilität erfordern können auch Bereiche sein, in denen eine erhöhte Flexibilität die natürliche Bewegung des Herzens unterstützt. Anpassungen können auch herbeigeführt werden durch die Wahl des verwendeten Materials, durch Veränderungen des Materials an bestimmten Bereichen durch Einwirkung von z.B. energetischer Strahlung, wie etwa Wärme. Vorzugsweise weist die Schale (2) Öffnungen auf, die von den Drähten eines Drahtgeflechtes, den Streben eines Gitterwerkes oder den Löchern in einem Schalenmantel geformt werden. Diese Öffnungen ermöglichen das Komprimieren der Schale (2), sie erlauben den Stoffaustausch vom Inneren der Schale (2) mit Bereichen außerhalb der Schale (2) und umgekehrt, sie verringern die Menge des einzusetzenden Materials für die Schale (2) und sie erlauben eine erhöhte Flexibilität der Schale (2). Formen, die mit durchgehenden Materialien nur schwer zu verwirklichen sind, sind mit geflechtartigen oder gitterartigen Strukturen leichter zu formen. Die Öffnungen können viereckig, rautenförmig, rund oder oval sein. Die durch die Drähte, die Streben oder den Löchern in einem Schalenmantel definierten Öffnungen haben einen Durchmesser von ungefähr 1 mm bis 50 mm, vorzugsweise von 3 mm bis 30 mm, vorzugsweise von 5 mm bis 20 mm. Der Durchmesser einer Öffnung ist als Pin-Öffnung definiert, das heißt der Durchmesser der Öffnung stellt den größten Durchmesser eines zylindrischen Stiftes dar, der durch eine Öffnung (eine Zelle, ein Loch) hindurchgeführt werden kann.

Die Schale (2) besteht vorzugsweise aus einem Material, das eine Expansion ermöglicht. Vorzugsweise ist die Schale (2) aus einem Material geformt, das aus der Gruppe bestehend aus Nitinol, Titan und Titanlegierungen, Tantal und Tantallegierungen, Edelstahl, Polyamid, Polymerfaser-Werkstoffe, Karbonfaserwerkstoffe, Aramidfaserwerkstoffe, Glasfaserwerkstoffe und Kombinationen daraus ausgewählt ist. Für eine selbst-expandierende Schale (2) eignet sich ein Material, das zumindest zum Teil aus einer Formgedächtnislegierung gefertigt ist. Werkstoffe für Formgedächtnislegierungen sind beispielsweise NiTi (Nickel-Titan; Nitinol), NiTiCu (Nickel-Titan-Kupfer), CuZn (Kupfer-Zink), CuZnAl (Kupfer-Zink-Aluminium), CuAINi (Kupfer-Aluminium-Nickel), FeNiAl (Eisen-Nickel-Aluminium) und FeMnSi (Eisen-Mangan-Silizium).

Die Schale (2) weist vorzugsweise eine an die individuelle Herzform des Patienten angepasste Form auf. Die individuelle Herzform der Patienten kann dabei aus CT- oder MRT-Bilddaten rekonstruiert werden. Die Schale (2) ist nach oben geöffnet. Der obere Rand der Schale (2) weist vorzugsweise Schlaufen eines Drahtes oder Bügel, die von Streben geformt sind, auf. Die Schlaufen oder Bügel können als Verankerungsstellen für eine Hülle (7) mit zumindest einer expandierbaren Einheit (71, 72) dienen. Am unteren Ende der becherförmigen Schale befindet sich vorzugsweise eine Öffnung, durch welche eine oder mehrere elektrische Leitungen (83) des zumindest einen Sensors (81) oder der zumindest einen Elektrode (82) und/oder Leitungen (41) der zumindest einen expandierbaren Einheit (71, 72) durchgeführt werden können. Die Form der Schale (2) stellt zumindest zum Teil die Form eines natürlichen Herzens (61) dar, vorzugsweise dem unteren Teil eines Herzens (61). Einzelheiten zur Form der Schale (2) werden in einem späteren Abschnitt der Beschreibung näher erläutert.

Die Schale (2) kann mit einer Membran (21) bedeckt sein, insbesondere mit einer Membran (21) aus Polyurethan, Silikon oder Polytetrafluorethylen (PTFE). Eine solche Membran kann den mechanischen Stress, der von der Schale (2) auf das Perikard (6) oder den Herzmuskel (61) ausgeübt wird, verringern. Ebenso kann eine derartige Membran (21) die Biokompatibilität der Schale (2) erhöhen oder das Anlagern von biologischem Gewebe und/oder Zellen verhindern oder verringern. Die Membran (21) kann auf der Innenseite oder auf der Außenseite der Schale (2) angebracht sein. Die Membran (21) kann auch durch Eintauchen der geflecht- oder gitterartigen Schale (2) in eine elastomerhaltige Flüssigkeit oder in flüssigen Kunststoff hergestellt werden und umkleidet dann das Gitter oder das Geflecht. Der Prozess kann dabei so eingestellt werden, dass die Öffnungen des Geflechts oder des Gitters von der Membran (21) bedeckt oder ausgespart werden können. Im letzten Fall würde die Membran dann nur die Streben des Gitterwerks oder die Drähte des Geflechts bedecken und nicht die Zwischenräume. Eine Membran (21) auf dem Geflecht oder dem Gitter kann auch das Widerlager für eine expandierbare Einheit (71, 72) verbessern. Ist beispielsweise eine expandierbare Einheit (71, 72) eine inflatierbare Kammer, so kann eine Membran (21) über, an oder auf dem Geflecht oder dem Gitter verhindern, dass während einer Expansion der Kammer Teile der Kammer durch das Geflecht oder das Gitter gedrückt werden. Die Membran (21) kann weiterhin eine zu große Aufweitung der Schale (2) verhindern, insbesondere bei Inflation einer inflatierbaren Kammer. Eine Membran (21) auf einem Gitter oder einem Geflecht kann gewährleisten, dass eine Expansion einer sich auf dem Gitter oder dem Geflecht befindlichen expandierbaren Einheit sich nur in eine vom Geflecht oder Gitter nach innen weisenden Richtung verläuft. Die Membran (21) behindert nicht die Komprimierbarkeit der Schale (2) beim Einbringen in ein Zuführsystem. Die Membran (21) kann 0,05 mm bis 0,5 mm dick sein, insbesondere wobei die Membran 0,1 mm bis 0,3 mm dick ist.

Die Schale (2) und/oder die Membran (21) kann auch einen Wirkstoff umfassen, beispielsweise einen antithrombotischen Wirkstoff, einen anti-proliferativen Wirkstoff, einen anti-inflammatorischen Wirkstoff, einen anti-neoplastischen Wirkstoff, einen anti-mitotischen Wirkstoff, einen anti-mikrobiellen Wirkstoff, einen Biofilm-Syntheseinhibitör, einen Antibiotika-Wirkstoff, einen Antikörper, einen antikoagulierenden Wirkstoff, einen cholesterol-senkenden Wirkstoff, einen beta-Blocker, oder eine Kombination davon. Vorzugsweise ist der Wirkstoff in Form einer Beschichtung auf der Schale (2) und/oder der Membran (21). Die Schale (2) und/oder Membran (21) kann auch mit extrazellulären Matrix-Proteinen, insbesondere Fibronektin oder Kollagen, beschichtet sein. Dies kann dann vorteilhaft sein, wenn ein Einwachsen der Schale (2) erwünscht ist.

In der Schale (2) befindet sich die zumindest eine expandierbare Einheit (71, 72). Figur 3 zeigt eine Schale (2), in die eine Hülle (7) mit expandierbaren Einheiten (71, 72) in der Form von inflatierbaren Kammern eingebracht ist. Die expandierbare Einheit (71, 72) wird durch eine Leitung (41) versorgt, die sich in dem Kabel (4) befindet. Die expandierbare Einheit (71, 72) kann eine hydraulische oder eine pneumatische Kammer sein. Die expandierbare Einheit (71, 72) kann direkt an der Schale (2) ohne eine Hülle (7) befestigt werden. Die expandierbare Einheit (71,72) kann auch auf einer Hülle (7) befestigt sein und die Hülle (7) kann in der Schale (2) befestigt sein. Die expandierbare Einheit (71, 72) kann ausgelegt sein, einen Druck auf das Herz (61) auszuüben. Dieser Druck kann ein permanenter Druck sein, oder er kann ein periodisch wiederkehrender Druck sein. Die erfindungsgemäße Vorrichtung kann verschiedene Arten von expandierbaren Einheiten (71, 72) umfassen. Die Vorrichtung kann zumindest eine Augmentationseinheit (71) umfassen. Die Vorrichtung kann zumindest eine Positionierungseinheit (72) umfassen. Die Augmentationseinheit (71) und/oder die Positionierungseinheit (72) können direkt an der Schale (2) angebracht werden oder auf einer Hülle (7), die in die Schale (2) eingebracht wird.

Eine Augmentationseinheit (71) ist eine Einheit, die periodisch expandiert und entspannt werden kann und so einen Druck auf das Herz (61) ausüben kann. Dieser Druck wird vorzugsweise in Bereichen auf den Herzmuskel ausgeübt, unter denen sich eine Herzkammer befindet. Durch Ausüben von Druck auf eine Herzkammer durch die Augmentationseinheit (71) wird die natürliche Pumpbewegung des Herzens (61) unterstützt oder ersetzt, und das Blut im Herzen (61) wird aus der Kammer in die abführende Arterie gepumpt. Ein von einer Augmentationseinheit (71) auf eine rechte Herzkammer ausgeübter Druck führt zum Auswurf des Blutes aus der rechten Herzkammer in die Lungenarterie. Ein von einer Augmentationseinheit (71) auf eine linke Herzkammer ausgeübter Druck führt zum Auswurf des Blutes aus der linken Herzkammer in die Aorta. Die Positionierung der zumindest einen Augmentationseinheit (71) in der Schale (2) wird in einem späteren Abschnitt der Beschreibung näher erläutert.

Eine Positionierungseinheit (72) ist eine Einheit die ebenfalls expandiert werden kann. Vorzugsweise wird eine Positionierungseinheit während des Betriebs der Vorrichtung zur Unterstützung der Funktion eines Herzens mehr statisch als periodisch expandiert. Die Positionierungseinheit (72) kann expandiert werden, um die Vorrichtung am Herzen zu fixieren und die Passform der Vorrichtung zu gewährleisten. Mit Hilfe einer Positionierungseinheit (72) kann auch auf Veränderungen des Herzmuskels reagiert werden (z.B. Schrumpfung des Herzmuskels aufgrund von Flüssigkeitsmangel oder Ausdehnung des Herzmuskels aufgrund Flüssigkeitsaufnahme des Patienten). Baut sich der Herzmuskel im Laufe eines Zeitraumes ab oder auf, kann eine Positionierungseinheit weiter expandiert und entspannt werden, um eine optimale Passform zu gewährleisten. Die Positionierungseinheit (72) kann beispielsweise auch dazu dienen, dass die Vorrichtung über die Dauer eines Herzschlages nicht den Kontakt mit der Herzwand verliert. Kontaktverlust kann zu Stoßbelastung zwischen dem Herzmuskel und der Vorrichtung führen und/oder Fehlfunktion von Sensoren (81) und/oder Elektroden (82) nach sich ziehen. Nicht abschließend kann die Positionierungseinheit (72) der pathologisch bedingten, progressiven Expansion des geschädigten Herzmuskels bei herzinsuffizienten Patienten entgegenwirken. Die Positionierung der zumindest einen Positionierungseinheit (72) in der Schale (2) wird in einem späteren Abschnitt der Beschreibung näher erläutert.

Am unteren Ende der Schale (2) kann sich eine Öffnung befinden, durch die die Leitung (83) von dem zumindest einen Sensor (81) oder der zumindest einen Elektrode (82) und/oder die Leitung (41) von der zumindest einen expandierbaren Einheit (71,72) durchgeführt werden kann. Die Öffnung kann am unteren distalen Ende der Schale (2) angebracht sein. Alternativ kann die Öffnung auch seitlich an der Schale (2) angebracht sein. Dargestellt ist in Figur 3 eine Öffnung am unteren distalen Ende der Schale (2), durch die ein Kabel (4), das sämtliche Leitungen (41, 83) umfasst, geführt ist. Es können auch mehrere getrennte Kabel anstelle eines Kabels (4) vorliegen. Die mehreren Kabel können durch eine Öffnung der Schale (2) oder durch mehrere Öffnungen der Schale (2) geführt werden. Am Ende des Kabels (4) ist ein Steckerteil (90) angebracht, mit Hilfe dessen der zumindest eine Sensor (81) oder die zumindest eine Elektrode (82) und/oder die zumindest eine expandierbare Einheit (71, 72) an eine Versorgungseinheit angeschlossen werden kann. Vorzugsweise wird die Schale (2) innerhalb des Perikards (6) angebracht. Das Kabel (4) wird dann durch das Perikard (6) geführt. Die erfindungsgemäße Vorrichtung kann einen Perikardverschluss (5) umfassen. Dieser kann die zur Kabeldurchführung notwendige Öffnung des Perikards (6) abdichten. Das Perikard (6) ist ein bindegewebiger Sack, der das Herz (61) umgibt und dem Herzen (61) durch eine schmale Gleitschicht freie Bewegungsmöglichkeit gibt. Es enthält als Gleitmittel eine seröse Flüssigkeit, die auch Liquor pericardii genannt wird. Damit dieses Gleitmittel nicht durch die Kabelöffnung aus dem Perikard (6) abfließen kann und damit keine anderen Flüssigkeiten oder Festkörper (wie z.B. Zellen, Proteine, Fremdkörper etc.) in das Perikard (6) gelangen können, kann ein Perikardverschluss (5) um das Kabel (4) angebracht werden. Der Perikardverschluss (5) dichtet die Öffnung des Perikards (6) zum Kabel (4) ab. Der Perikardverschluss (5) kann ein erstes Verschlusselement mit einer ersten Dichtlippe und ein zweites Verschlusselement mit einer zweiten Dichtlippe umfassen. Durch ein zentrales Lumen des Verschlusses kann ein Kabel (4) durchgeführt werden. Die erste Dichtlippe und/oder die zweite Dichtlippe können die Perikardöffnung abdichten. Im zentralen Lumen kann sich ein zusätzliches Dichtelement befinden, das das Kabel (4) gegen den Perikardverschluss (5) abdichten und gegebenenfalls auch fixieren kann. Das erste und das zweite Verschlusselement können gekoppelt werden. Vorzugsweise können das erste und das zweite Verschlusselement mit einem Mechanismus gesichert werden. Mögliche Mechanismen zur Sicherung der Verschlusselemente sind Schraubmechanismen, Klemmmechanismen oder ein Bajonettverschluss. Das erste Verschlusselement und/oder das zweite Verschlusselement können expandierbar sein, vorzugsweise selbst-expandierbar sein. Der Perikardverschluss (5) wird in einem späteren Abschnitt der Beschreibung näher erläutert.

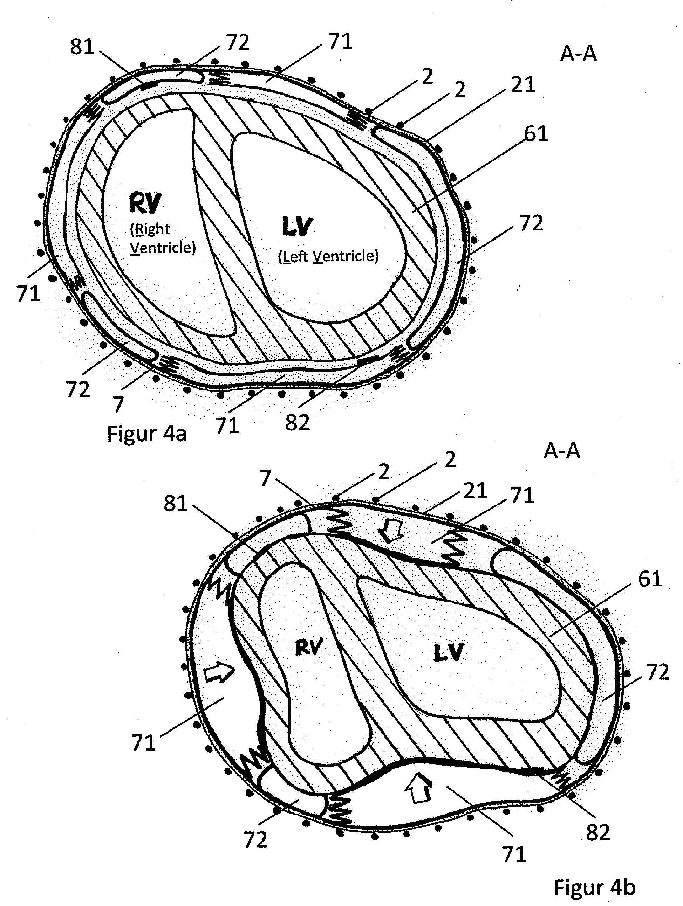

**Figuren 4a und 4b** zeigen ein Schnitt durch das Herz (61) und einen Teil der Vorrichtung zur Unterstützung der Funktion eines Herzens (61) entlang der Linie A-A in Figur 3. Von außen nach innen sind folgende Schichten dargestellt: Die Schale (2) mit einer Membran (21), eine Hülle (7) mit zumindest einer expandierbaren Einheit (71, 72), einen Sensor (81), eine Elektrode (82) und das Herz (60) im Transversalschnitt. Exemplarisch sind drei Augmentationseinheiten (71) und drei Positionierungseinheiten (72) eingezeichnet. In Figur 4a sind die expandierbaren Einheiten (71, 72) im nicht-expandierten Zustand eingezeichnet. In Figur 4b sind die Augmentationseinheiten (71) in einem expandierten Zustand eingezeichnet. Die zumindest eine expandierbare Einheit (71, 72) befindet sich in einem einer Herzkammer benachbarten Bereich. Eine Expansion der zumindest einen expandierbaren Einheit (71, 72) kann das Volumen der Herzkammer verkleinern und führt damit zu einem Auswurf von Blut aus der Kammer. Der zumindest eine Sensor (81) oder die zumindest eine Elektrode (82) ist an einer Stelle angebracht, an der zumindest ein Parameter des Herzens (61) gemessen werden kann. Eine Elektrode (82) kann an einer Stelle angebracht sein, an der der Herzmuskel stimuliert werden kann. In den Figuren 4a und 4b sind exemplarisch ein Sensor (81) und eine Elektrode (82) auf den expandierbaren Einheiten (71,72) eingezeichnet. Die Sensoren (81) und/oder die Elektrode kann auch in der in der Wand einer expandierbaren Einheit (71,72), auf der Innenseite oder Außenseite der Hülle (7), in der Hülle (7) oder an der Schale (2) angebracht sein. Es können auch zwei, drei, vier, fünf oder mehr Sensoren und/oder Elektroden auf der Hülle angebracht sein.

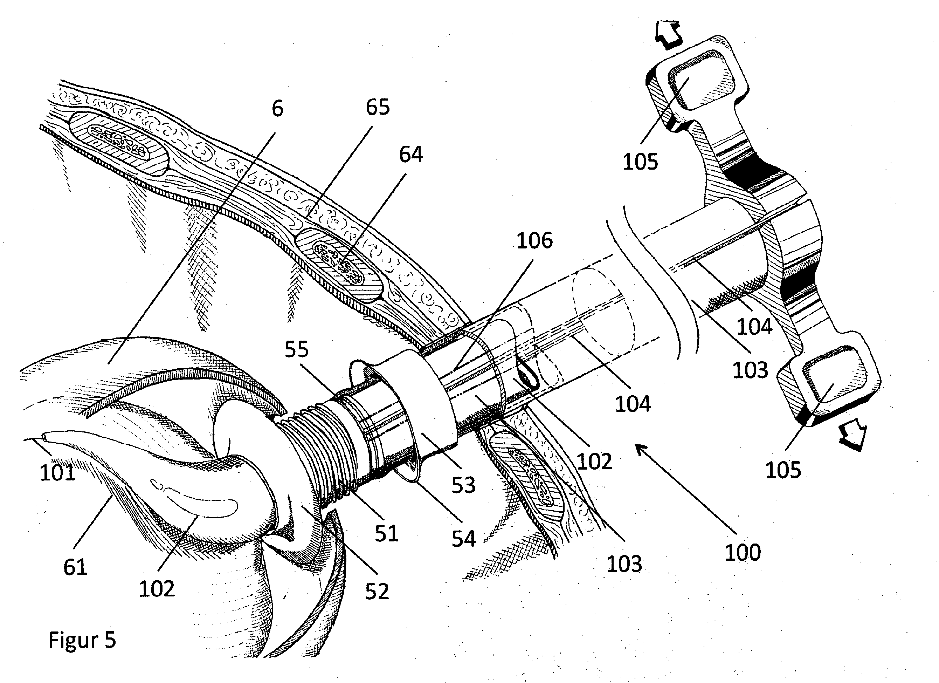

**Figur 5** zeigt ein Zuführsystem (100) mit Hilfe dessen die Vorrichtung zur Unterstützung der Funktion eines Herzens implantiert werden kann. Das Zuführsystem (100) besteht aus einem Katheter (103), der ein Lumen hat. Vorzugsweise ist der Katheter (103) ein längliches röhrenförmiges Element, in den die Vorrichtung zur Unterstützung der Funktion eines Herzens in einem komprimierten Zustand eingebracht werden kann. Der Querschnitt des Katheter (103) und/oder des Lumens kann rund, oval oder polygonal sein. Das Zuführsystem (100) kann ferner einen Führungsdraht (101) und/oder ein Dilatationselement umfassen. Das Dilatationselement kann eine weiche kegelförmige Spitze (102) mit Schaft sein. Durch eine Punktur der Brustwand (65) zwischen den Rippen (64) und des Perikards (6) kann der Führungsdraht (101) geführt werden. Die weiche kegelförmige Spitze (102) kann in der Mitte ein rundes, ovales oder polygonales Lumen haben. Die weiche kegelförmige Spitze (102) kann über den Führungsdraht (101) geschoben werden und die Punktur kann dilatiert werden ohne das Epikard zu verletzen. Durch die dilatierte Öffnung kann der distale Abschnitt des Katheters (103) des Zuführsystems (100) hindurchgeführt werden. Am distalen Ende des Katheters (103) kann ein erstes Verschlusselement (51, 52) des Perikardverschlusses aufgesteckt oder anderweitig befestigt sein. Beispielsweise kann der Katheter (103) auf einen am Ende des ersten Verschlusselements (51, 52) befindlichen Konus (55) aufgesteckt sein. Nicht dargestellt ist eine andere Ausführungsform, bei der sich ein Konus auf der Seite des Katheters befindet und das erste Verschlusselement dann auf diesen aufgesteckt werden kann. Der Katheter (103) mit dem befestigten ersten Verschlusselement (51, 52) des Perikardverschlusses kann über den Schaft der weichen Spitze (102) geführt und in das Perikard (6) eingebracht werden. Alternativ können der Katheter (103) und das erste Verschlusselement (51, 52) des Perikardverschlusses nicht miteinander gekoppelte Teile sein. In diesem Fall wird zunächst nur der Katheter (103) in das Perikard (6) eingeführt und das erste Verschlusselement (51, 52) kann dann über den Katheter in das Perikard (6) geschoben oder durch das Lumen des Katheters (103) im Perikard (6) ausgebracht werden. Das erste Verschlusselement (51, 52) kann ein selbst-expandierbares Verschlusselement sein und kann sich im Perikard (6) entfalten. Alternativ enthält ein nicht-expandierbares Teil (51) des ersten Verschlusselements eine selbstexpandierbare Dichtlippe (52) oder eine Dichtlippe (52), welche sich beim Einführen des ersten Verschlusselements (51, 52) anlegen kann und sich im Perikard (6) aufspreizt. Das erste Verschlusselement (51, 52) kann zu einer pilzförmigen oder regenschirmartigen Form expandieren. Ein zweites Verschlusselement (53, 54) kann über den Katheter (103) eingeführt werden oder durch den Katheter (103) ausgebracht werden. Beispielsweise kann das zweite Verschlusselement (53, 54) über den Katheter (103) des Zuführsystems (100) zum distalen Ende des Zuführsystems (100) bewegt werden und kann dann mit dem ersten Verschlusselement (51, 52) gekoppelt werden. Das zweite Verschlusselement (53, 54) kann expandierbar oder nicht-expandierbar sein. Das zweite Verschlusselement (53, 54) kann mit dem ersten Verschlusselement (51,52) koppelbar sein. Vorzugsweise ist das zweite Verschlusselement (51, 52) selbst-expandierbar und kann in einer expandierten Form eine pilzförmige oder regenschirmartige Form annehmen. Das zweite Verschlusselement (53, 54) kann mit dem ersten Verschlusselement (51, 52) gesichert werden. Dargestellt in Figur 5 ist ein Schraubmechanismus. Andere Mechanismen zur Sicherung der Verschlusselemente (51, 52, 53, 54) umfassen einen Klemmmechanismus oder einen Bajonettverschluss. Nach dem Sichern der Verschlusselemente (51, 52, 53, 54) kann der Katheter (103) des Zuführsystems (100) noch am Konus (55) des ersten Verschlusselements (51) verbleiben oder im Lumen des Verschlusselementes (51, 52) verbleiben. Nachdem der Führungsdraht (101) und die weiche Spitze mit Schaft (102) aus dem Katheter herausgezogen wurden, kann die Schale mit dem zumindest einen Sensor oder der zumindest einen Elektrode und/oder mit der zumindest einen expandierbaren Einheit durch das Lumen des Katheters (103) eingebracht werden. Die Schale ist vorzugsweise selbst-expandierend und umschließt nach Expansion das Herz (61) zumindest zum Teil. Am unteren Ende der Schale kann sich ein Steckerteil oder ein Kabel mit einem Steckerteil befinden. Die Versorgungseinheit kann direkt an der Schale befestigt sein oder über ein Kabel mit der Schale verbunden werden. Nach dem Zuführen der Schale kann das Zuführsystem (100) entfernt werden. Dafür kann sich am Zuführsystem (100) und/oder am Katheter (103) eine Sollbruchstelle (104) befinden. Vorzugsweise befindet sich eine oder mehrere Sollbruchstellen (104) entlang einer longitudinalen Achse des Zuführsystems (100). Die Sollbruchstelle (104) kann eine Sollbruchlinie darstellen. Wird das Zuführsystem (100) entlang einer Sollbruchstelle (103) aufgebrochen, kann das Zuführsystem (100) geteilt, entrollt und entfernt werden. Das Zuführsystem (100) kann auch Greifelemente (105) umfassen, mit Hilfe derer eine Kraft auf das Zuführsystem (100) ausgeübt werden kann. Vorzugsweise kann mit den Greifelementen (105) eine seitwärts vom Katheter (103) des Zuführsystems (100) gerichtete Kraft ausgeübt werden, die geeignet ist, die Sollbruchstelle (104) aufzubrechen.

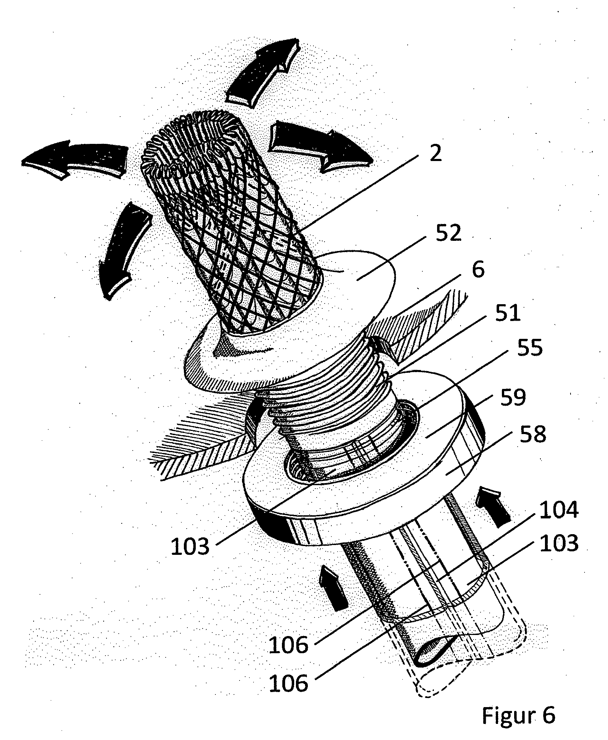

**Figur 6** zeigt einen Schritt der Implantation der erfindungsgemäßen Vorrichtung. Nachdem das erste Verschlusselement (51, 52) im Perikard (6) die expandierte Form eingenommen hat, kann die Schale (2), die vorzugsweise selbst-expandierend ist, durch das Lumen des Katheters (103) des Zuführsystems und das Lumen des ersten Verschlusselementes (51) hindurchgeführt werden. Nachdem sich zumindest ein Teil der Schale (2) mit dem zumindest einen Sensor oder der zumindest einen Elektrode und/oder der zumindest einen expandierbaren Einheit im Perikard (6) befindet, beginnt die Expansion der Schale (2). Dargestellt in Figur 6 ist auch das zweite Verschlusselement (58, 59) bevor es mit dem ersten Verschlusselement (51, 52) gekoppelt wurde. Das zweite Verschlusselement (58, 59) ist in diesem Ausführungsbeispiel ein ringförmiges Element (58), z.B. eine Mutter, an dessen distaler Seite eine Dichtscheibe (59) angebracht sein kann. Das zweite Verschlusselement (58, 59) kann expandierbar oder nicht expandierbar sein. Das zweite Verschlusselement (58, 59) kann auf dem Katheter (103) verschoben werden. In diesem Ausführungsbeispiel besitzen das erste (51, 52) und das zweite Verschlusselement (58, 59) Gewindeabschnitte die miteinander verschraubt werden können.

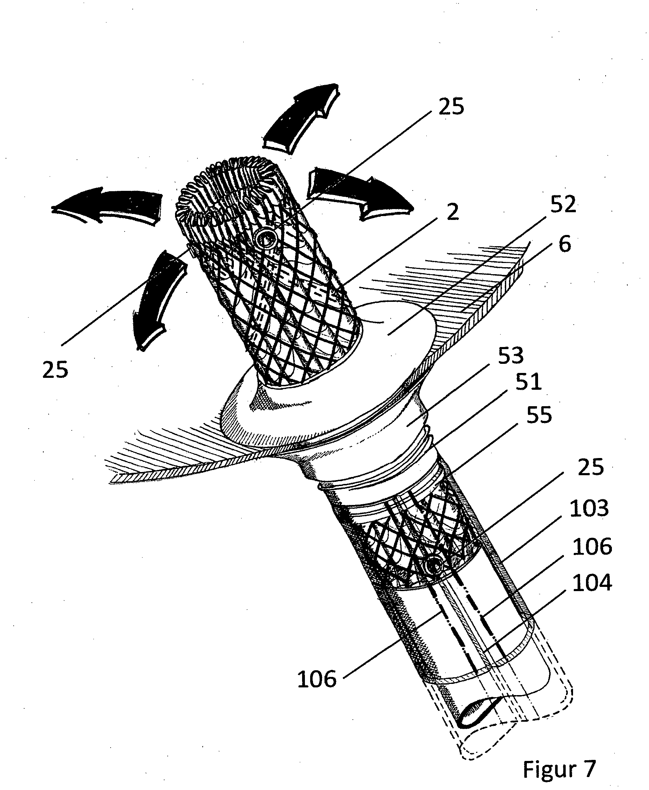

**Figur 7** zeigt einen Schritt der Implantation der erfindungsgemäßen Vorrichtung. In diesem Ausführungsbeispiel ist das erste Verschlusselement (51, 52) mit dem zweiten Verschlusselement (53) gekoppelt. Das Perikard (6) kann dadurch abgedichtet sein. Die expandierbare Schale (2) befindet sich teilweise im Perikard (6) und kann expandiert werden. Figur 7 zeigt Markierungen (25), die auf der Schale (2) angebracht sind. Die erfindungsgemäße Vorrichtung enthält im Allgemeinen zumindest eine Markierung (25), die ein korrektes Platzieren der Schale (2) erleichtern kann. Die Markierung (25) kann eine optische Markierung sein, insbesondere eine Farbmarkierung. Die Markierung (25) kann eine phosphoreszierende oder fluoreszierende Markierung sein, die deren Wahrnehmung in einer dunklen Umgebung erleichtert. Solche Umgebungen können im Operationssaal selbst sein und können unter anderem durch Schattenwurf verursacht werden. Solche Umgebungen können auch im Körperinneren eines Patienten sein. Die Markierung (25) kann aus einem Material gefertigt sein, das mit Hilfe bildgebender Verfahren dargestellt werden kann. Geeignete bildgebende Verfahren umfassen Röntgenverfahren, CT-Verfahren, und MRT-Verfahren. Die Markierung (25) kann aus einem röntgendichteren Material geformt sein als das Material benachbarter Regionen. Die Markierung (25) kann eine Markierung in der Form eines Punktes, eines Kreises, eines Ovals, eines Polygons oder die Form eines Buchstabens annehmen. Andere Formen können durch Verbinden von Punkten erzeugte Flächen sein. Beispielsweise kann die Form ein Halbmond oder ein Stern sein. Die Markierung (25) kann auf der Schale (2) angebracht sein oder auf einer Hülle angebracht sein. Die Markierung kann in Form einer Linie angebracht sein. Die Linie kann von einem oberen Rand der Schale (2) ausgehen. Die Linie kann von einem oberen Rand der Schale (2) zu einem Punkt an der unteren Spitze der Schale (2) verlaufen. Die Linie kann vom oberen Rand der Schale lotrecht zur unteren Spitze der Schale (2) verlaufen. Der Ausgangspunkt der Linie am oberen Rand der Schale (2) kann sich an einer Stelle befinden, die im implantierten Zustand nahe eines oder an einem Bereich in Höhe des Septums des Herzens ist. Die Markierung (25) kann sich an Kreuzungspunkten des Geflechts oder des Gitters befinden. Besteht die Schale (2) aus einem Schalenmantel, in den Löcher geformt wurden, kann die Markierung (25) in den Schalenmantel eingearbeitet werden. Beispielsweise kann ein Loch mit einer vordefinierten Form gefertigt werden, das dann als Markierung (25) dient.

Das Zuführsystem und/oder der Katheter (103) des Zuführsystems kann eine oder mehrere Markierungen (106) umfassen. Eine Markierung (106) auf einem Zuführsystem kann wie eine Markierung auf einer Schale geformt sein. Die Markierung (106) kann die Form eines Punktes oder die Form einer Linie haben. Eine Markierung (106) in Form einer Linie kann eine zumindest zum Teil einen Umfang des Zuführsystems beschreibende Linie sein. Eine Markierung (106) in Form einer Linie kann eine longitudinale Linie entlang einer Achse des Zuführsystems sein. Eine Markierung (106) in Form einer Linie kann eine gerade Linie oder eine mäandernde Linie sein. Eine Markierung (106) in Form einer Linie kann eine auf einem Katheter (103) eines Zuführsystems schräg verlaufende Linie sein. Eine Markierung (103) kann die Orientierung des Zuführsystems bei der Implantation erleichtern. Eine Markierung (103) an oder auf dem Zuführsystem kann in Deckung mit einer Linie an oder auf einem medizinischen Implantate sein. Beispielsweise kann das medizinische Implantat eine Vorrichtung zur Unterstützung der Funktion eines Herzens sein, die komprimiert werden kann. In einem komprimierten Zustand kann die Vorrichtung in ein Zuführsystem eingebracht werden. Eine oder mehrere Markierungen (25) auf oder an der Vorrichtung können mit einer oder mehreren Markierungen (106) auf oder an dem Zuführsystem in Deckung gebracht werden. Derartige Markierungen (25, 106) erleichtern die Orientierung eines medizinischen Implantates. Markierungen (25) können sich auch entlang einer Achse eines medizinischen Implantates befinden. Derartige Markierungen (25) können hilfreich sein, um den Fortschritt des Ausbringens eines medizinischen Implantates aus einem Zuführsystem zu verfolgen. Das Zuführsystem und/oder ein Katheter (103) kann aus einem transparenten Material gefertigt sein, das es erlaubt das medizinische Implantat während dem Einführen wahrnehmen zu können.

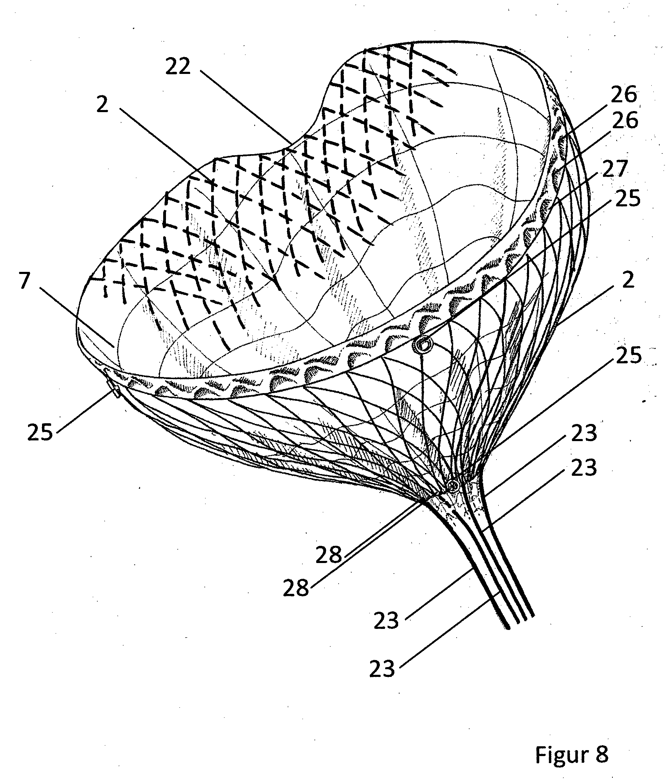

**Figur 8** zeigt die vollständig expandierte Konfiguration der Schale (2) und einer Hülle (7) der erfiridungsgemäßen Vorrichtung. In diesem Beispiel sind keine expandierbaren Einheiten und keine Zuleitungen eingezeichnet. Dargestellt ist eine selbst-expandierende Schale (2). Die Schale (2) ist in diesem Ausführungsbeispiel aus einem Gitterwerk oder Drahtgeflecht geformt, das am oberen Rand und/oder am unteren Rand der Schale (2) Streben in Form von Bügeln oder Schlaufen (26, 28) aufweist. Ist die Schale (2) aus einem Schalenmantel geformt sein, in den Löcher geformt wurden, kann die Schale (2) am oberen Rand und/oder am unteren Rand derart gestaltet sein, dass sich zumindest ein Bügel am oberen Rand und/oder am unteren Rand der Schale (2) befindet. Die in Figur 8 dargestellte Schale (2) umfasst eine Hülle (7), die in die Schale (2) eingebracht ist. Auf der Hülle (7) und der Schale (2) kann sich zumindest eine expandierbare Einheit befinden. Die Hüllen können an den Schlaufen (26, 28) oder Bügeln der Schale (2) befestigt werden. Insbesondere kann eine Hülle an den Schlaufen (26, 28) oder Bügeln der Schale (2) eingehängt werden. In einem solchen Fall kann die Hülle (7) zumindest eine Tasche (27) aufweisen, die über zumindest eine Schlaufe (26, 28) oder zumindest einen Bügel gestülpt werden kann. Eine andere Ausführungsform kann eine Hülle (7) umfassen, die über die Schale (2) ragt und welche dann am oberen Rand der Schale (2) um die Bügel/Schlaufen (26) und/oder am unteren Rand umgestülpt wird. Der umgestülpte Teil der Hülle (7) kann durch das Gitterwerk oder das Drahtgeflecht hindurch mit dem nicht umgestülpten Teil der Hülle verbunden werden. Beispielsweise kann die Umstülpung durch das Gitterwerk oder das Drahtgeflecht hindurch verschweißt oder verklebt werden. Diese Umstülpung kann auch eine um die gesamte oder um einen Teil der Hülle (7) umlaufende Tasche (27) bilden, die in den oberen Rand und/oder den unteren Rand der Schale (2) eingehängt werden kann. Die Befestigung durch Umstülpen eines Teils der Hülle um den oberen Rand der Schale ermöglicht ein passgenaues Fixieren der Hülle in der Schale. Durch das Umstülpen der Hülle um den oberen Rand der Schale wird auch das Gewebe vor möglichen Beschädigungen durch den oberen Rand der Schale geschützt. Die Hülle (7) kann aus Kunststoff, Polymer, Kautschuk, Gummi, Latex, Silikon oder Polyurethan bestehen. Die Hülle (7) kann eine Dicke von 0,1 mm bis 1 mm, vorzugsweise 0,2 mm bis 0,5 mm aufweisen.

Die Schale (2) kann am unteren Ende eine Öffnung haben. Ein Teil der Hülle (7) kann durch diese Öffnung durchragen, beispielsweise können zwischen 3 mm und 2 cm der Hülle durchragen. Die Hülle (7) kann ebenfalls unten geöffnet sein, womit der Flüssigkeitsaustausch vom Raum innerhalb von Hülle (7) zum Raum zwischen Schale (2) und Perikard (6) verbessert wird. So kann beispielsweise Perikardflüssigkeit durch die Öffnung in der Hülle (7) ein- und ausfließen.

Der obere Rand der Schale (2) verläuft bevorzugt parallel zur Herzklappenebene, kann aber Bereiche einer Aussparung (22) aufweisen. Die Aussparung (22) in der Schale (2) kann aus anatomischen Gegebenheiten erforderlich sein, beispielsweise um die Vena cava inferior nicht zu kompromittieren, welche von dorsal in Richtung des rechten Vorhofs des Herzens verläuft. Eine Kompromittierung der Vena cava inferior würde zu einer unteren Einflussstauung führen (Behinderung der Befüllung des rechten Vorhofs). Eine Aussparung (22) kann auch aufgrund anderer anatomischer oder kardialer Strukturen erforderlich sein. Wird die Schale (2) als Gitterwerk oder Drahtgeflecht ausgeführt, so kann die Aussparung (22) durch nachträgliches Abtrennen von Streben oder Drähten hergestellt werden. Alternativ kann auch ein Fertigungsverfahren gewählt werden, bei dem die Aussparung (22) nicht im Nachhinein hergestellt werden muss. So kann zum Beispiel bei einer aus einem Drahtgeflecht hergestellten Schale die Drahtwicklung so angepasst werden, dass die Drähte an der Stelle der Aussparung (22) kürzer gewählt werden und die Kreuzungspunkte näher zusammenrücken. Bei einer Schale (2), die aus einem Gitterwerk besteht, das aus einem geschlitzten Rohr ("slotted tube") gefertigt wurde, kann die Aussparung (2) durch geeigneten Zuschnitt des Rohres und entsprechend modifizierte Schlitzung angefertigt werden. Die Schlitzung kann so angepasst werden, dass trotz der Aussparung (22) die Anzahl der Zellen in der Schale (2) gleich bleibt. Das kann aus Stabilitätsgründen erforderlich sein. Wird mehr Flexibilität in der Schale (2) gewünscht, so kann die Schlitzung des Rohres derart modifiziert werden, dass an der Stelle der Aussparung (22) eine, zwei oder drei Zellreihen wegfallen. Die Hülle (7) kann an die Aussparung (22) angepasst werden. Entlang des oberen Schalenrandes kann die Aussparung eine Länge von 2 cm, 3 cm, 4 cm, 5 cm, 6 cm, 7 cm oder mehr haben. Die Tiefe der Aussparung, gemessen vom gedachten nicht-unterbrochenen oberen Schalenrand zum Punkt der Aussparung welcher der Herzspitze am nächsten liegt, kann zwischen 1 mm und 40 mm betragen, insbesondere zwischen 3 mm und 15 mm. In Umfangsrichtung ist die Aussparung dort positioniert, wo die Vena cava inferior in den rechten Vorhof mündet. Die Aussparung kann bogenförmig, halbkreisförmig, rechteckig oder polygonal sein. Die Aussparung (22) kann so lang sein, dass sie dem Umfang des oberen Randes der Schale (2) entspricht. Die Aussparung (22) kann dann an dem Punkt des oberen Randes der Schale (2) beginnen und enden, welcher der auszusparenden kardialen Struktur, beispielsweise der Vena cava inferior gegenüberliegt. In diesem Fall kann resultieren, dass die Aussparung (22) eine Ebene definiert, welche zur Herzklappebene verkippt ist und welche an der auszusparenden Stelle 1 mm bis 40 mm, insbesondere 3 mm bis 15 mm tiefer zu liegen kommt, als wenn der obere Rand der Schale (2) parallel zur Herzklappenebene verlaufen würde. Die Hülle (7) weist eine Aussparung (22) auf, die in ihrer Form, Lage und Dimension im Wesentlichen mit der Aussparung (22) in der Schale (2) übereinstimmt, welche den anatomischen Bereich um die Vena cava inferior freistellt. Eine Kompromittierung der Vena cava inferior durch die Hülle (7) und eine folgliche Behinderung der Befüllung des rechten Vorhofs wird dadurch verhindert. Die Aussparungen (22) in Hülle (7) und Schale (2) können miteinander in Deckung gebracht werden.

In Figur 8 weist die Schale (2) mehrere Markierungen (25) auf. Wie bereits beschrieben können diese Markierungen (25) verschiedene Formen oder Positionen einnehmen. Im vorliegenden Fall sind die Markierungen (25) an einem oberen Rand und an der unteren Spitze der Schale (2) angebracht.

Am unteren Ende der Schale (2) können eine, zwei, drei, vier oder mehr Verlängerungsstreben (23) an der Schale angebracht werden. Die Verlängerungsstreben stellen eine Verlängerung der Schale (2) in Richtung der Herzlängsachse dar. Die Verlängerungsstreben (23) können in Richtung einer gedachten Herzspitze verlaufen, sich dort berühren oder nicht ganz berühren und von dort aus in Richtung der Herzlängsachse vom Herzen wegragen. Die Verlängerungsstreben (23) können in einer gemeinsamen Hülse gesammelt werden. Die Verlängerungsstreben und/oder die Hülse können mit dem Kabel der Vorrichtung verbunden werden, beispielsweise durch Kleben. Die Verlängerungsstreben (23) können dazu dienen die Schale in axialer Richtung zu fixieren. Die Verlängerungsstreben (23) schränken die Rotation der Schale um die Herzlängsachse nur bedingt ein. Ist die Schale aus einem Drahtgeflecht gefertigt, so können die Verlängerungsstreben (23) die verlängerten Enden von Drähten darstellen. Die Verlängerungsstreben (23) und das Drahtgeflecht sind somit ein Teil. Besteht die Schale (2) aus einem Gitterwerk, das aus einem geschlitzten Rohr ("slotted tube") gefertigt wurde, so kann das Gitterwerk mit den Verlängerungsstreben (23) durch geeignete Schnittführung aus nur einem Rohr gefertigt werden. Das Gitterwerk und die Verlängerungsstreben (23) sind somit ein Teil. Die Verlängerungsstreben (23) und die Schale (2) können auch mehrteilig sein, womit die Verlängerungsstreben (23) nach der Fertigung der Schale (2) an dieser befestigt werden, beispielsweise durch einen Einhängemechanismus oder durch Stoffschluss (Anschweißen, Ankleben). Die Länge der Verlängerungsstreben (23) kann zwischen 1cm und 10cm, insbesondere zwischen 4cm und 7cm betragen.

Die Schale (2) kann nur die oben beschriebene Aussparung (22) und keine Verlängerungsstreben (23) umfassen. Alternativ kann die Schale (2) sowohl die oben beschriebene Aussparung (22) und die oben beschriebenen Verlängerungsstreben (23) umfassen. Die Schale (2) kann auch nur die oben beschriebenen Verlängerungsstreben (23) und keine Aussparung (22) umfassen.

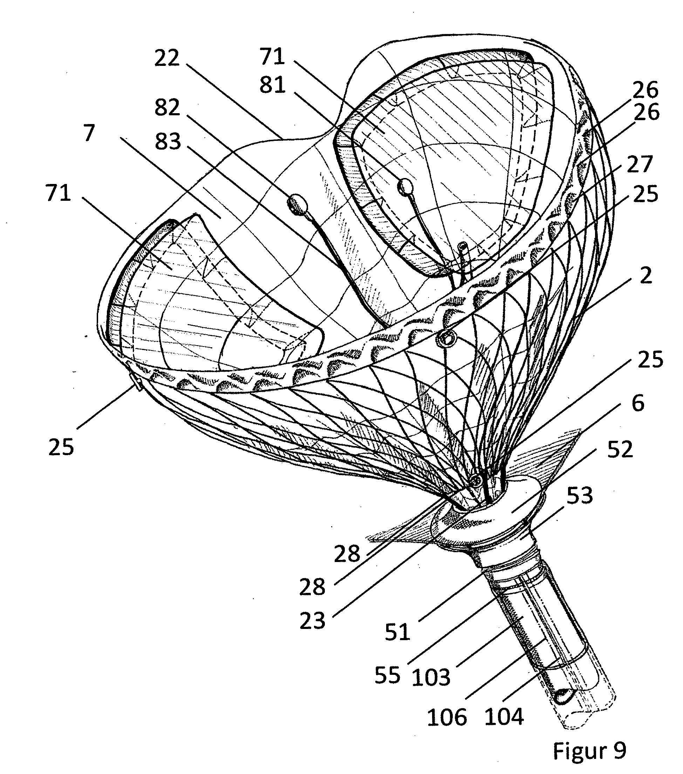

**Figur 9** zeigt einen Schritt der Implantation der erfindungsgemäßen Vorrichtung. Aus Gründen der Übersichtlichkeit ist das Herz nicht mit dargestellt. In diesem Beispiel sind das erste Verschlusselement (51,52) und das zweite Verschlusselement (53) des Perikardverschlusses miteinander gekoppelt. Die Vorrichtung zur Unterstützung der Funktion eines Herzens wurde bereits zu einem Teil aus dem Zuführsystem ausgebracht. Dargestellt ist eine selbst-expandierende Schale (2) in die eine Hülle (7) eingebracht wurde. Auf der Hülle sind expandierbare Einheiten (71) in Form balgförmigen Kammern, ein Sensor (81) und eine Elektrode (82) angebracht. Die Schale (2) und die Hülle in diesem Ausführungsbeispiel gleichen im Wesentlichen jeweils der Schale und der Hülle aus Figur 8.

Auf die in Figur 9 dargestellte Hülle (7) wurden in diesem Ausführungsbeispiel noch expandierbare Einheiten (71) aufgebracht. Die Hülle (7) kann die Unterseite der expandierbaren Einheiten (71) sein. Die expandierbaren Einheiten (71) können auf die Form des Herzens, der Schale (2) und/oder der Hülle (7) angepasst sein. Auf die Hülle (7) können eine, zwei, drei, vier, fünf, sechs, sieben oder mehr expandierbare Einheiten (71) aufgebracht werden. Beim Expandieren können die expandierbaren Einheiten (71) einen Druck auf den Herzmuskel ausüben. Dieser Druck wird vorzugsweise in Bereichen auf den Herzmuskel ausgeübt, unter denen sich eine Herzkammer befindet, jedoch immer unterhalb der Herzklappenebene. Werden die expandierbaren Einheiten (71) im unteren Bereich der Hülle (7) und/oder Schale (2) nahe an der Herzspitze, positioniert, so wird bei der Expansion der expandierbaren Einheiten (71) der Herzmuskel in Richtung der Herzlängsachse nach oben zur Herzklappenebene gedrückt. Werden die expandierbaren Einheiten (71) im oberen Bereich der Hülle (7) und der Schale (2) angeordnet, so wird bei Expansion der expandierbaren Einheiten (71) der Herzmuskel senkrecht zur Herzlängsachse gedrückt. Auf den Herzmuskel wird dann parallel zur Herzklappenebene (seitlich auf die Ventrikel) Druck ausgeübt. Die expandierbaren Einheiten (71) können auch an jeder Position zwischen dem unteren Rand und dem oberen Rand von Hülle (7) und Schale (2) positioniert werden, womit der Druck der expandierbaren Einheiten (71) bei deren Expansion anteilig in Richtung der Herzlängsachse und anteilig parallel zur Herzklappenebene wirkt. In Figur 9 sind expandierbare Einheiten (71) dargestellt die sich am oberen Rand der Hülle (7) befinden. Bei Expansion der expandierbaren Einheiten (71) wirkt der Druck daher möglichst parallel zur Herzklappenebene. Die Gegenkraft auf die Vorrichtung in Richtung der Herzlängsachse wird dadurch gering gehalten. Eine Verschiebung der Vorrichtung in Richtung der Herzlängsachse vom Herzen weg (Dislokation), verursacht durch Expansion der expandierbaren Einheiten (71), wird dadurch minimiert.

Die selbst-expandierende Schale (2) und die Hülle (7) weisen in dieser Darstellung eine Aussparung (22) auf um die Vena cava inferior im implantierten Zustand der Vorrichtung nicht zu kompromittieren. Die expandierbaren Einheiten (71) können in ihrer Größe, Form und Position an die Aussparung (22) in der Schale (2) und der Hülle (7) angepasst werden, so dass sie weder im expandierten, noch im nicht-expandierten Zustand die Aussparung (22) überdecken.

Am unteren Ende der Schale (2) befinden sich Verlängerungsstreben (23), welche die Schale (2) in axialer Richtung zum Kabel fixieren und die Vorrichtung im gesamten damit stabilisieren. Die Verlängerungsstreben (23) können mit dem Kabel der Vorrichtung verbunden sein. Ein Teil der Verlängerungsstreben (23) kann auch nach der Implantation im Perikardverschluss oder außerhalb der Verschlusselemente (51, 52, 53) des Perikardverschlusses verbleiben. Würde durch die Expansion der expandierbaren Einheiten (71) die Vorrichtung eine Kraft in Richtung der Herzlängsachse vom Herzen weg erfahren, so kann diese Kraft zum Teil über die Schale (2), die Verlängerungsstreben (23) und die Zuleitung (Kabel) abgetragen werden, ohne dass sich eine Dislokation der Vorrichtung ergibt. Die Verlängerungsstreben können (23) an deren Enden Löcher, Bügel, Schlaufen oder abgewinkelte Enden haben, die eine minimal-invasive Explantation der Schale (2) und der Hülle (7) erleichtern. Die abgewinkelten Enden, die Löcher, Bügel oder Schlaufen behindern nicht die Fixierung der Verlängerungsstreben (23) im Kabel. Bei der Implantation oder während des Betriebs der Vorrichtung können sich Enden der Verlängerungsstreben (23) innerhalb des Kabels befinden. Bei der Explantation der Vorrichtung kann das Kabel etwa auf Höhe der Enden der Verlängerungsstreben (23) abgeschnitten werden und die Schale (2) mit der Hülle (7) an den Enden der Verlängerungsstreben (23) durch die Verschlusselemente (51, 52, 53) aus dem Perikard (6) herausgezogen werden. Zur Explantation der Schale (2) und der Hülle (7) kann ein Werkzeug an die abgewinkelten Enden der Verlängerungsstreben (23) oder die an den Enden der Verlängerungsstreben (23) befindlichen Löcher, Bügel oder Schlaufen gekoppelt werden.

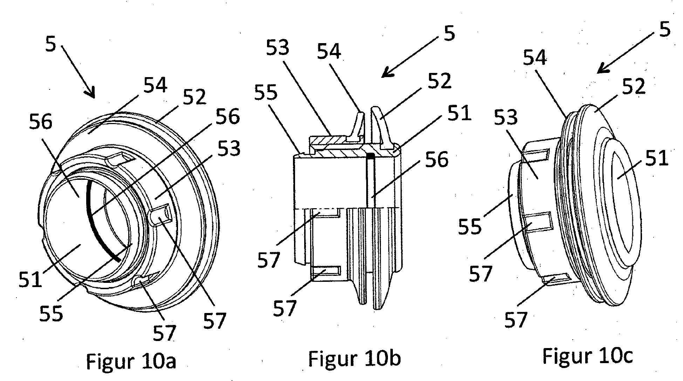

**Figuren 10a****-c** zeigen verschiedene Ansichten eines Perikardverschlusses (5). Der Perikardverschluss (5) dient der Vermeidung von Verlust von Perikardflüssigkeit, oder auch der Möglichkeit künstliche Perikardflüssigkeit, auch mit der Zugabe von Medikamenten oder andere Therapeutika applizieren zu können. Die Vermeidung von Verlust von Perikardflüssigkeit dient auch der Vermeidung von Verwachsungen des Systems mit dem Epikard. Der Perikardverschluss (5) besteht im Allgemeinen aus einem ersten Verschlusselement (51) und einem zweiten Verschlusselement (52). Das erste Verschlusselement (51) hat ein zentrales Lumen und das zweite Verschlusselement (53) hat ein zentrales Lumen. Das erste Verschlusselement (51) kann mit dem zweiten Verschlusselement (53) koppelbar sein. Nach dem Koppeln des ersten Verschlusselements (51) mit dem zweiten Verschlusselement (52) weist der gekoppelte Perikardverschluss (5) ein durch den Perikarverschluss (5) verlaufendes Lumen auf. Das Lumen kann ausschließlich vom zentralen Lumen des ersten Verschlusselements (51) gebildet werden oder das Lumen kann ausschließlich vom zentralen Lumen des zweiten Verschlusselements (53) geformt sein. Das Lumen kann in einer anderen Ausführungsform auch aus den beiden Lumen der beiden gekoppelten Verschlusselemente (51,53) geformt sein. In dem Lumen kann sich eine Dichtungsmanschette, ein O-Ring, eine Labyrinth-Dichtung oder eine anderes Dichtungselement (56) befinden. Ein Dichtungselement (56) im Lumen des Perikardverschlusses kann den Perikardverschluss (5) gegen ein durch den Perikardverschluss (5) ragendes Objekt abdichten. Beispielsweise kann ein Kabel durch den Perikardverschluss (5) geführt werden, das dann gegen den Perikardverschluss (5) abgedichtet ist. Ein Dichtungselement (56) im Lumen kann nicht nur dem Abdichten, sondern auch dem Fixieren eines durch das Lumen des Perikardverschlusses ragenden Objekts dienen. Das Dichtungselement (56) kann auf beiden Verschlusselementen (51, 53) oder nur an einem der beiden Verschlusselemente (51, 53) angebracht sein.

Das erste Verschlusselement (51) kann mit dem zweiten Verschlusselement (53) mit Hilfe eines Mechanismus gesichert werden. Ein Mechanismus zum Sichern eines ersten Verschlusselements (51) mit einem zweiten Verschlusselement (53) kann einen Schraubmechanismus oder einen Klemmmechanismus umfassen. Ein Mechanismus zum Sichern eines ersten Verschlusselements (51) mit einem zweiten Verschlusselement (53) kann auch einen Bajonettverschluss umfassen. Das erste Verschlusselement (51) und das zweite Verschlusselement (53) können aus dem gleichen Material oder aus verschiedenen Materialien gefertigt sein. Geeignete Materialien für das erste Verschlusselement (51) und/oder das zweite Verschlusselement (53) umfassen Kunststoffe, Metalle, Keramiken oder Kombinationen davon.

Am ersten Verschlusselement (51) kann eine erste Dichtlippe (52) angebracht sein. Die erste Dichtlippe (52) kann Teil des ersten Verschlusselements (51) sein oder sie kann an das erste Verschlusselement (51) angebracht werden. Am zweiten Verschlusselement (53) kann eine zweite Dichtlippe (54) angebracht sein. Die zweite Dichtlippe (54) kann Teil des zweiten Verschlusselements (53) sein oder sie kann an das zweite Verschlusselement (53) angebracht werden. Die erste Dichtlippe (52) und die zweite Dichtlippe (54) können aus dem gleichen Material oder aus verschiedenen Materialien geformt sein. Eine oder beide Dichtlippen (52, 54) können Teil des jeweiligen Verschlusselementes (51, 53) sein und aus dem gleichen Material wie das zugehörige Verschlusselement (51, 53) geformt sein. Die erste Dichtlippe (52) und/oder die zweite Dichtlippe (54) können aus Kunststoff (vorzugsweise aus einem Elastomer), Kautschuk, Gummi, Silikon, Latex oder einer Kombination davon geformt sein. Die erste Dichtlippe (52) und/oder die zweite Dichtlippe (54) können scheibenförmig sein. Die erste Dichtlippe (52) und/oder die zweite Dichtlippe (54) können eine konkave oder konvexe Biegung aufweisen. Gebogene Dichtlippen (52, 54) können sich besser an die anatomischen Gegebenheiten anpassen. Das Perikard weist im Bereich der Herzspitze eine konvexe Form auf. Indem die Dichtlippen (52, 54) eine Biegung in Gestalt der anatomisch vorliegenden Form des Perikards aufweisen, kann ein verbesserter anatomischer Sitz des Perikardverschlusses (5) erreicht werden. Ebenfalls können mit gebogenen Dichtlippen (52, 54) verbesserte Dichteigenschaften erreicht werden. Die erste Dichtlippe (52) und/oder die zweite Dichtlippe (54) können Verstärkungen aufweisen. Mit zunehmendem radialem Abstand vom Lumen des Perikardverschlusses nach außen kann die erste Dichtlippe (52) und/oder die zweite Dichtlippe (54) eine erhöhte Flexibilität aufweisen. Eine erhöhte Flexibilität in Randbereichen einer Dichtlippe (52, 54) kann die Dichteigenschaften der Dichtlippe (52, 54) und auch die anatomische korrekte Positionierung der Dichtlippe (52, 54) fördern. Eine erhöhte Flexibilität in Randbereichen einer Dichtlippe (52, 54) kann durch die Materialwahl erreicht werden. Jede der Dichtlippen (52, 54) kann aus einem Material oder aus mehreren Materialien bestehen. Verstärkungen in einer Dichtlippe (52, 54) können konzentrische Verstärkungen sein oder radiale Verstärkungen. Verstärkungen können erreicht werden durch veränderliche Materialstärken oder durch Einbringen eines verstärkenden Materials. Das verstärkende Material kann das gleiche Material sein wie das Grundmaterial der Dichtlippe (52, 54), das in eine andere Form des Materials überführt wurde. Alternativ können Regionen die nicht verstärkt sein sollen geschwächt werden durch Überführen des Materials der Dichtlippe (52, 54) in eine schwächere Form des Materials. Eine Schwächung des Materials kann durch Einwirkung von energetischer Strahlung, wie z.B. Wärme, herbeigeführt werden. Verstärkungen des Materials können auch erreicht werden durch Aufbringen von Material, wobei das aufgebrachte Material das gleiche Material wie das Grundmaterial der Dichtlippe (52, 54) darstellen kann, oder wobei das aufgebrachte Material ein anderes Material als das Grundmaterial der Dichtlippe (52, 54) darstellen kann. Geeignete Materialien zur Verstärkung von Teilen einer Dichtlippe (52, 54) sind Metalle, Keramiken, Gummi und oder eine Kombination davon.

An einem der beiden Verschlusselemente (51, 53) kann sich ein Kopplungsmechanismus befinden, der das Koppeln eines Verschlusselementes (51, 53) mit dem Zuführsystem oder einem Katheter des Zuführsystems ermöglicht. Der Kopplungsmechanismus kann beispielsweise aus einem Konus (55) bestehen der sich am ersten Verschlusselement (51) befindet auf den das Zuführsystem oder ein Katheter eines Zuführsystems geklemmt werden kann. Die Klemmwirkung kann beispielsweise erreicht werden indem der Durchmesser des Konus (55) größer ist als der luminale Durchmesser des Zuführsystems. Der Kopplungsmechanismus zur Kopplung des Perikardverschlusses (5) mit dem Zuführsystem, kann sich auch am zweiten Verschlusselement (53) befinden. Der Kopplungsmechanismus kann auch als separates Bauteil neben den Verschlusselementen (51, 53) vorliegen und das Zuführsystems mit einem der beiden Verschlusselemente (51, 53) des Perikardverschlusses (5) verbinden. Andere Ausführungsformen des Kopplungsmechanismus umfassen unter anderem einen nicht-konischen (beispielsweise zylindrischen) Fortsatz an einem der Verschlusselemente (51, 53) auf den das Zuführsystem aufgebracht oder aufgeklebt werden kann oder auch eine Ausführung bei welcher der Katheter des Zuführsystems und ein Verschlusselement ein einzelnes Integralteil darstellen. In der letztgenannten Ausführung kann nach erfolgreicher Einbringung und Sicherung des Perikardverschlusses (5) der Katheter mittels einer Sollbruchstelle vom Verschlusselement (51,53) des Perikardverschlusses (5) getrennt werden. An einem oder an beiden Verschlusselementen (51, 53) können sich Eingreifelemente (57) befinden. Diese Eingreifelemente (57) können genutzt werden, um eine Kraft auf eines oder auf beide Verschlusselemente (51, 53) auszuüben, die geeignet ist die Verschlusselemente (51, 53) zu koppeln und/oder zu sichern. Eingreifelemente (57) auf einem oder auf beiden Verschlusselementen (51, 53) können Löcher, Einbuchtungen oder Erhebungen sein. Die Eingreifelemente (57) können umlaufend um das Verschlusselement (51, 53) in gleichem Abstand voneinander angebracht werden. Der umlaufende Abstand zwischen den Eingreifelementen (57) kann auch verschieden sein. In den Figuren 10a-c sind sechs Eingreifelemente (57) in gleichmäßigem umlaufenden Abstand voneinander dargestellt. Auf dem ringförmigen Verschlusselement (53) sind die sechs Eingreifelemente (57) in einem Winkelabstand von ca. 60° angebracht. Bei zwei, drei, vier, fünf, sechs, acht oder mehr gleichmäßig angeordneten Eingreifelementen (57) kann der Winkelabstand respektive 180°, 120°, 90°, 72°, 60°, 45° oder weniger betragen. Die Eingreifelemente (57) können auch unregelmäßig angeordnet werden.

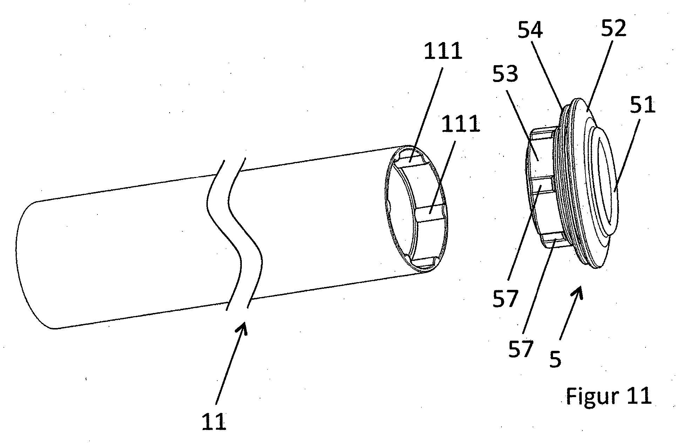

**Figur 11** zeigt einen Perikardverschluss (5) und ein Werkzeug (11) zum Sichern eines Perikardverschlusses (5). Der in Figur 11 dargestellte Perikardverschluss (5) stimmt im Wesentlichen mit dem in Figur 10 gezeigten Verschluss überein. Das Werkzeug (11) ist exemplarisch als längliches röhrenförmiges Werkzeug dargestellt. Am distalen Ende des Werkzeuges (11) befinden sich Elemente (111) die zumindest teilweise mit den Eingreifelementen (57) eines Verschlusselements (53) in Eingriff gebracht werden können. In dem in Figur 11 gezeigten Ausführungsbeispiel befinden sich in dem röhrenförmigen Werkzeug (11) am distalen Ende sechs nach innen zeigende Erhebungen (111) die mit den sechs Eingreifelementen (57) des Verschlusselementes (53), in diesem Fall sechs Einbuchtungen auf dem Verschlusselement (53), in Eingriff gebracht werden können. Das Werkzeug (11) weist im Wesentlichen die gleiche Anzahl an Elementen (11) auf, die komplementär zu den Eingreifelementen (57) des Verschlusselementes (53) sind. Das in Figur 11 dargestellte Werkzeug (11) ist ein röhrenförmiges Werkzeug, das aus einer vollständig umlaufenden Röhre besteht. Das röhrenförmige Element des Werkzeugs (11) kann auch eine Halbröhre, Viertelröhre, oder eine Drittelröhre sein. Im Extremfall kann anstatt der Röhre nur ein Stiel oder mehrere Stiele an einem distalen ringförmigen Werkzeug angebracht sein. Ein Stiel kann sich von dem ringförmigen Werkzeug in longitudinaler Richtung erstrecken. Ein Stiel kann sich auch seitlich weg von einer longitudinalen Achse des Werkzeug erstrecken. Andere ebenfalls nicht gezeigte Ausführungsformen des Werkzeuges (11) können in Form eines modifizierten Ringschlüssels oder eines modifizierten Gabelschlüssels vorliegen.

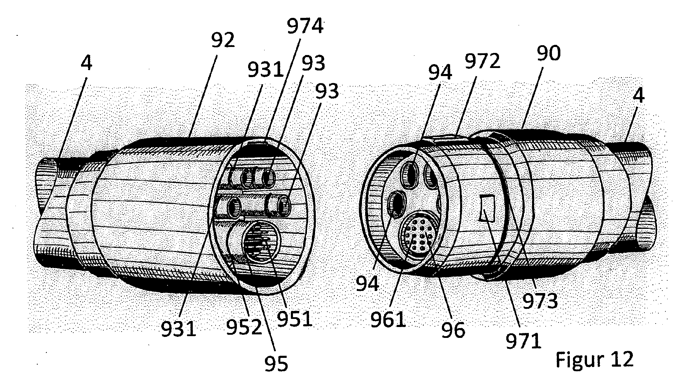

**Figur 12** zeigt ein Steckersystem bestehend aus zwei Steckerteilen (90, 92). Die Vorrichtung zur Unterstützung der Funktion eines Herzens besteht aus einer Schale mit zumindest einem Sensor oder zumindest einer Elektrode und/oder zumindest einer expandierbaren Einheit, wobei der zumindest eine Sensor oder Elektrode und/oder die zumindest eine expandierbare Einheit mit einer Versorgungseinheit verbunden ist. Der zumindest eine Sensor oder die zumindest eine Elektrode und/oder die zumindest eine expandierbare Einheit kann direkt mit der Versorgungseinheit verbunden werden. Der zumindest eine Sensor oder die zumindest eine Elektrode und/oder die zumindest eine expandierbare Einheit kann über ein Kabel (4) mit der Versorgungseinheit verbunden werden. Der zumindest eine Sensor oder die zumindest eine Elektrode und/oder die zumindest eine expandierbare Einheit kann über das Kabel (4) direkt mit der Versorgungseinheit verbunden sein oder der zumindest eine Sensor oder die zumindest eine Elektrode und/oder die zumindest eine expandierbare Einheit kann an die Versorgungseinheit angeschlossen sein. Die Versorgungseinheit kann einen Steckerteil (92) umfassen. Das Steckerteil (92) kann direkt an der Versorgungseinheit angebracht sein. Das Steckerteil (92) kann über ein Kabel (4) mit der Versorgungseinheit verbunden sein. Der zumindest eine Sensor oder die zumindest eine Elektrode und/oder die zumindest eine expandierbare Einheit können ein Kabel (4) umfassen. Am Ende des Kabels (4) kann ein Steckerteil (90) sein. Das Steckerteil (90) am Ende des Kabels des zumindest einen Sensors oder der zumindest einen Elektrode und/oder der zumindest einen expandierbaren Einheit kann mit dem Steckerteil (92) der Versorgungseinheit gekoppelt werden. Das Steckerteil (90) des Sensors oder der Elektrode und/oder der zumindest einen expandierbaren Einheit kann ein männliches Steckerteil oder ein weibliches Steckerteil sein. Ein weibliches Steckerteil auf der Seite des Sensors oder der Elektrode und/oder der zumindest einen expandierbaren Einheit kann vorteilhaft sein, da das weibliche Steckerteil im Gegensatz zum männlichen Steckerteil keine Pins (951) oder sonstige Anschlüsse umfasst, die hervorragen und damit brechen können. Ist der Austausch der Versorgungseinheit notwendig muss das Steckersystem entkoppelt werden und eine neue Versorgungseinheit in den Steckerteil (90) des Sensors oder der Elektrode und/oder der zumindest einen expandierbaren Einheit eingekoppelt werden. Hierbei kann es zum Bruch von Pins (951) oder sonstigen Anschlüssen kommen. Sind diese Pins (951) oder Anschlüsse auf einem männlichen Steckerteil auf der Seite der Schale mit dem zumindest einen Sensor oder der zumindest einen Elektrode und/oder der zumindest einen expandierbaren Einheit, kann ein Austausch der Schale erforderlich sein. Ein weibliches Steckerteil auf der Seite der Schale mit dem zumindest einen Sensor oder der zumindest einen Elektrode und/oder der zumindest einen expandierbaren Einheit kann vorteilhaft sein, da ein Brechen von Pins (951) oder sonstigen Anschlüssen an einem weiblichen Steckerteil nicht vorkommen kann. Das Steckersystem (90, 92) besteht im Allgemeinen aus zwei Steckerteilen. Die erfindungsgemäße Vorrichtung kann aus einem Steckersystem (90, 92) für den zumindest einen Sensor oder die zumindest eine Elektrode und/oder die zumindest einen expandierbaren Einheit bestehen oder aus mehreren Steckersystemen. Werden mehrere Steckersysteme verwendet, kann ein Steckersystem für elektrische Leitungen und ein Steckersystem für hydraulische und/oder pneumatische Leitungen vorhanden sein. Das in Figur 12 dargestellte Steckersystem (90, 92) ist ein Steckersystem das Anschlüsse zur Versorgung von dem zumindest einen Sensor oder der zumindest einen Elektrode und der zumindest einen expandierbaren Einheit umfasst. Die Anzahl der Anschlüsse hängt davon ab, wie viele Sensoren oder Elektroden und wie viele expandierbare Einheiten verwendet werden, wobei die Anzahl nicht direkt mit der Anzahl der Sensoren oder Elektroden und/oder der Anzahl der expandierbaren Einheiten korrelieren muss. Gabelungen von Leitungen sind auf beiden Seiten des Steckersystems (90, 92) möglich und eine pneumatische oder hydraulische Leitung kann ein, zwei, drei, vier, fünf, sechs oder mehr befüllbare Kammern versorgen. Die Befüllung der mehreren Kammern durch eine Leitung muss nicht parallel erfolgen, sie kann auch individuell durch steuerbare Ventile erfolgen. Ebenso kann eine elektrische Leitung im Kabel für mehrere Sensoren oder Elektroden verwendet werden und Schalter können individuell Schaltkreise ansteuern. Das in Figur 12 dargestellte Steckersystem (90, 92) umfasst vier hydraulische oder pneumatische Anschlüsse (93, 94) und einen Anschluss für elektrische Leitungen (95, 96). Der in Figur 12 gezeigte Anschluss für elektrische Leitungen (95, 96) weist 16 Anschlusselemente in Form von Pins (951) und Pin-Buchsen (961) auf. Mehr oder weniger Anschlüsse für elektrische Leitungen (95, 96) und/oder pneumatische oder hydraulische Leitungen (93, 94) können in einem Steckersystem vorliegen. Es können ein, zwei, drei, vier, fünf, sechs, sieben, acht, neun oder zehn Anschlüsse für pneumatische oder hydraulische Leitungen (93, 94) vorliegen. Es können ein, zwei, drei, vier, fünf, sechs, sieben, acht, neun, zehn, zwölf, vierzehn, sechszehn, zwanzig oder mehr Anschlüsse für elektrische Leitungen (95, 96) vorliegen. Es können ein, zwei, drei, vier, fünf, sechs, sieben, acht, neun, zehn, zwölf, vierzehn, sechszehn, zwanzig oder mehr Anschlusselemente in Form von Pins (951) und Pin-Buchsen (961) innerhalb eines elektrischen Anschlusses für elektrische Leitungen (95, 96) vorliegen. Die Anzahl der Anschlusselemente in Form von Pins (951) und Pin-Buchsen (961) ist aber für das jeweilige Anschlusspaar für elektrische Leitungen (95,96) identisch. Jeder der Anschlüsse (93, 94, 95, 96) in einem oder in beiden der Steckerteile des Steckersystems (90, 92) kann eine eigene Dichtung (931, 952) haben. Die Dichtung (931, 952) der einzelnen Anschlüsse (93, 94, 95, 96) kann ein Dichtungsband oder ein Dichtungsring sein. Das Steckersystem (90, 92) kann zusätzlich oder nur eine Dichtung im Inneren des Steckersystems (973) oder um das Steckersystem herum haben. Eine Dichtung über das Steckersystem kann ein Dichtungsband oder ein Dichtungsring sein. Die Steckerteile (90, 92) können miteinander gekoppelt werden um das Steckersystem (90, 92) zu bilden. Die Steckerteile (90, 92) können auch eine Führungsnase (972) und eine Führungsnut (974) besitzen. Die Führungsnase (972) und die Führungsnut (974) können ein falsches Koppeln der beiden Steckerteile und/oder ein Verdrehen der Steckerteile während des Koppelvorgangs verhindern. Es können auch zwei, drei oder mehr Führungsnasen (972) und Führungsnuten (974) vorliegen. Im Falle von zwei oder mehr Führungsnasen (974) und Führungsnuten (974) werden ungleiche Abstände zwischen den einzelnen Führungsnasen (974) und Führungsnuten (974) verwendet. Die gekoppelten Steckerteile (90, 92) können auch mit einem Mechanismus (971) gesichert werden. Ein solcher Mechanismus (971) kann ein Schraubmechanismus oder ein Klemmmechanismus oder ein Bajonettverschluss sein. Ein Mechanismus zum Sichern des gekoppelten Steckersystems (90, 92) kann auch eine außen am Steckersystem angebrachte Überwurfmutter, Klemme, Riegel oder Schnapparretierung sein. Das Sichern des Steckersystems (90, 92) ist vorteilhaft, da ein versehentliches oder zufälliges teilweises oder gesamtes Öffnen des Steckersystems (90, 92) die Versorgung des zumindest einen Sensors oder der zumindest einen Elektrode und/oder der zumindest einen expandierbaren Einheit unterbrechen kann.

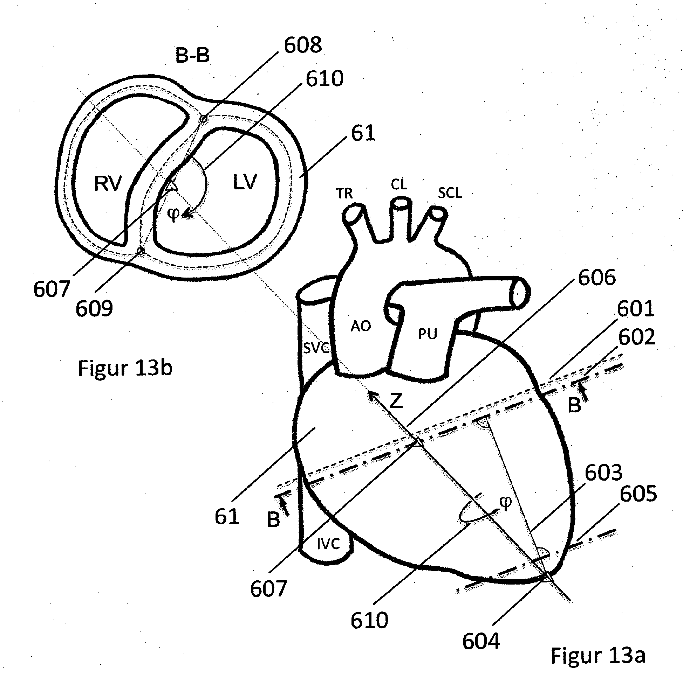

**Figur 13** zeigt ein Modell zum Erstellen eines Koordinatensystems und zur Dimensionierung von Schale und Hülle. Das Erstellen eines Koordinatensystems kann das Herstellen einer Vorrichtung zur Unterstützung der Funktion eines Herzens erleichtern, da Dimensionen von Hülle und Schale, die Position für den zumindest einen Sensor oder eine Elektrode und/oder die zumindest eine expandierbare Einheit und/oder die zumindest eine Markierung exakt definiert werden können.

Figur 13a zeigt ein Herz (61) mit anatomischen Orientierungspunkten. Dargestellt ist das Herz (61) mit dem von der linken Herzkammer (LV) ausgehende Aortenbogen (AO) (mit den abzweigenden Kopf-, Hals- und Schlüsselbeinarterien (TR, CL, SCL)) und die von der rechten Herzkammer (RV) ausgehende Lungenarterie (PU). Ebenso dargestellt sind Teile der unteren Hohlvene (IVC, Vena cava inferior) und der oberen Hohlvene (SVC). Die gestrichelt dargestellte Linie (601) stellt die Lage der Klappenebene dar. Durch Fällen des Lotes (603) von dieser Ebene (601) durch den am weitesten distal liegenden Punkt der Herzspitze wird der Punkt (604) der Herzspitze definiert. Alle diese Orientierungspunkte oder ein vollständiges 3D-Abbild einer individuellen Herzform können dabei aus CT- oder MRT-Bilddaten eines Patienten rekonstruiert werden. Zur Ermittlung der Orientierungspunkte oder zur Erstellung des 3D-Abbildes des Herzens können CT- oder MRT-Bilddaten verwendet werden die zu bestimmten Zeitpunkten während eines Herzzyklus aufgenommen wurden (EKG-getriggerte Bilddaten-Akquisition). Hierbei bieten sich Bilddaten an, die während der Diastole des Herzzyklus aufgenommen wurden, insbesondere zu einem Zeitpunkt zwischen 50 % und 100 % des zeitlichen Verlaufs der Diastole, insbesondere zwischen 75 % und 80 %, insbesondere bei 78 % des zeitlichen Verlaufs der Diastole. Es können auch Bilddaten verwendet werden die zu einem Zeitpunkt während der Systole des Herzzyklus oder ohne EKG-Triggerung aufgenommen wurden. Die erfindungsgemäße Vorrichtung besteht aus einer Schale in die eine Hülle mit zumindest einem Sensor oder einer Elektrode und/oder eine Hülle mit zumindest einer expandierbaren Einheit eingebracht werden kann. Die Dimensionierung der Schale und/oder der Hülle kann auf dem aus CT- oder MRT-Bilddaten rekonstruierten 3D-Abbild des Herzens basieren. Die Schale und/oder Hülle kann dabei kleiner, genauso groß oder größer als das 3D-Abbild des Herzens sein. Die Schale und/oder Hülle kann einen Abstand zur Oberfläche des 3D-Abbildes haben oder zur Oberfläche des 3D-Abbildes skaliert werden. Die Oberfläche des 3D-Abbildes kann dem Epikard oder dem Perikard des reellen Herzens entsprechen. Die Schale kann einen konstanten Abstand von 1 mm bis 10 mm zum 3D-Abbild aufweisen, insbesondere 2 mm bis 8 mm, insbesondere 3 mm bis 5 mm. Anstelle eines konstanten Abstandes kann der Abstand zwischen dem 3D-Abbild und der Schale auch lokal innerhalb der Grenzen von 1 mm und 10 mm variieren, insbesondere von 2 mm bis 8 mm, insbesondere von 3 mm bis 5 mm. Die Größe der Schale kann alternativ durch räumliche Streckung des 3D-Abbildes der Herzoberfläche festgelegt werden, insbesondere wobei der räumliche Streckungsfaktor in einem Bereich von 1,01 bis 1,2 liegt. Die Hülle kann einen konstanten Abstand von 1 mm bis 7 mm zum 3D-Abbild aufweisen, insbesondere 2 mm bis 5 mm.

Anstelle eines konstanten Abstandes kann der Abstand zwischen dem 3D-Abbild und der Hülle auch lokal innerhalb der Grenzen von 1 mm und 7 mm variieren, insbesondere von 2 mm bis 5 mm. Die Größe der Hülle kann alternativ durch räumliche Streckung des 3D-Abbildes der Herzoberfläche festgelegt werden, insbesondere wobei der räumliche Streckungsfaktor in einem Bereich von 1,004 bis 1,1 liegt, vorzugsweise in einem Bereich von 1,005 bis 1,06. Der obere Rand der Schale und/oder Hülle (602) kann parallel zur Klappenebene verlaufen mit einem in Richtung der Herzspitze nach unten versetzten Abstand von der Klappenebene von 1 mm bis 30 mm, 3 mm bis 20 mm, 5 mm bis 10 mm, vorzugsweise 5 mm. Der obere Rand der Schale und/oder Hülle ist in Figur 13a mit der Linie (602) dargestellt. Der untere Rand der Schale und/oder Hülle (605) kann parallel zur Klappenebene mit einem Abstand zu dem am meisten distal liegenden Punkt (604) von 1 mm bis 30 mm, 3 mm bis 20 mm, 5 mm bis 10 mm, vorzugsweise 5 mm sein.

Figur 13b zeigt eine Schnittebene B-B entlang der in Figur 13a dargestellten Linie (602), also entlang der Linie, die dem oberen Rand der Schale entspricht. In Figur 13b sind die rechte Herzkammer (rechter Ventrikel, RV) und die linke Herzkammer (linker Ventrikel, LV), die Herzwand und die Septumswand, die die Herzkammern trennt, gezeigt. Die Punkte (608) und (609) sind definiert als die Kreuzungspunkte der Mittellinien der Herzwand mit der Septumswand. Der Punkt (608) wird auch als anteriorer Kreuzungspunkt der Mittellinien der Herzwand mit der Septumswand bezeichnet. Der Punkt (609) wird auch als posteriorer Kreuzungspunkt der Mittellinien der Herzwand mit der Septumswand bezeichnet. Der Mittelpunkt auf einer Linie, die die Punkte (608) und (609) verbindet, wird als Punkt (607) definiert. Ein polares Koordinatensystem kann mit Hilfe dieser Punkte definiert werden. Die z-Achse (606) des polaren Koordinatensystems ist definiert als die Verbindungslinie des am meisten distal liegenden Punkt (604) mit dem Mittelpunkt (607) der Linie, die die Punkte (608) mit (609) verbindet. Die Umfangsrichtung des Koordinatensystems ist mit dem Bezugszeichen (610) angedeutet und ist als Winkelmaß ϕ definiert, wobei eine von der z-Achse (606) radial durch den anterioren Kreuzungspunkt (608) verlaufende Linie als ϕ=0° definiert ist. Mit Hilfe des Koordinatensystems können die Position für den zumindest einen Sensor oder eine Elektrode und/oder die zumindest eine expandierbare Einheit und/oder die zumindest eine Markierung definiert werden.

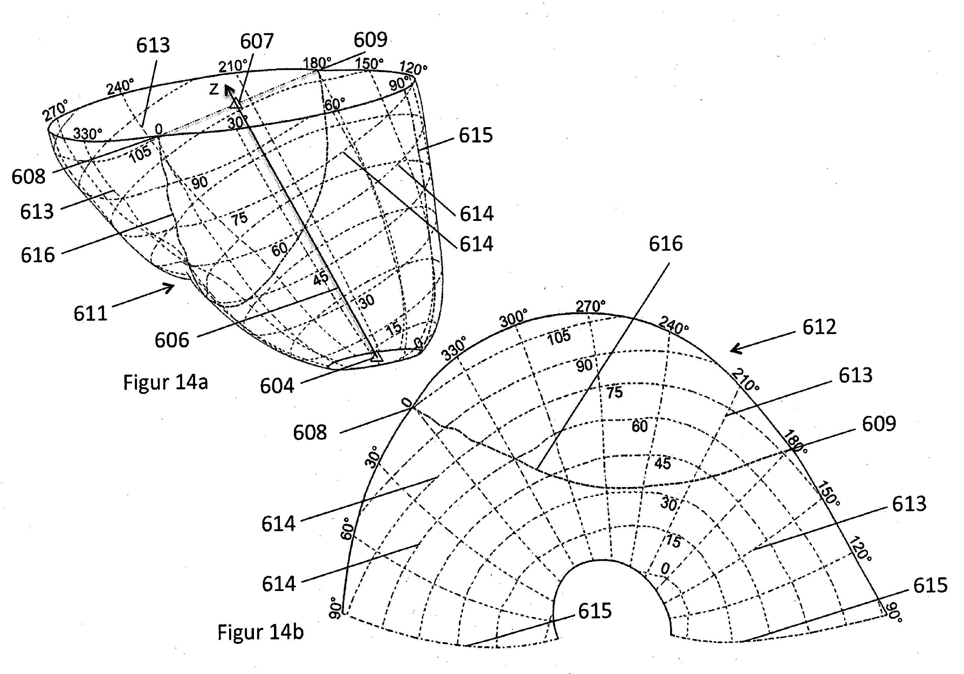

**Figur 14** zeigt eine Schale und/oder Hülle mit dem oben in Zusammenhang mit Figur 13 beschriebenen Koordinatensystem. Figur 14a zeigt ein 3D-Modell (611) einer Schale oder Hülle mit der z-Achse (606), die durch den am meisten distal liegenden Punkt (604) und den Mittelpunkt (607) der Linie, die die Punkte (608) mit (609) verbindet. Die Punkte (608) bzw. (609) sind der anteriore bzw. posteriore Kreuzungspunkt der Mittellinien der Herzwand mit der Septumswand, wobei durch den Punkt (608) die ϕ=0° Linie gelegt wird. Die gestrichelte Linie, die die Punkte (608) mit (609) entlang eines äußeren Umfangs der Schale oder Hülle verbindet, stellt die auf die Schale/Hülle projizierte Lage der Septumswand des Herzens dar. Am oberen Rand der Schale oder Hülle sind die Winkelmaße von ϕ=0° ausgehend in 30° Schritten angezeigt, wobei die Winkel von oben gesehen gegen den Uhrzeigersinn ansteigen. Auf die Schale/Hülle projizierte longitudinale Linien (613) verlaufen entlang dieser Winkelangaben jeweils bis zu Herzspitze (604). Das Winkelmaß von ϕ=360° entspricht dann wieder dem Winkelmaß von ϕ=0°. Höhenlinien (614) sind mit Abständen von jeweils 15 mm Schritten eingezeichnet. Die Höhenlinien (614) und Ebenen verlaufen lotrecht zur z-Achse (606). Die gepunktet-gestrichelte Linie (615) stellt eine Schnittlinie dar, an der die 3D-Form (611) aufgeschnitten und dann entrollt werden kann. Figur 14b zeigt eine entrollte Schale oder Hülle (612), die entlang der Linie (615) in Figur 14a geschnitten wurde und dann ausgerollt wurde. Die in Figur 14b dargestellten Positionen (608, 609) und Linien (613, 614, 615, 616) repräsentieren die gleichen Positionen und Linien, die in Figur 14a dargestellt sind.

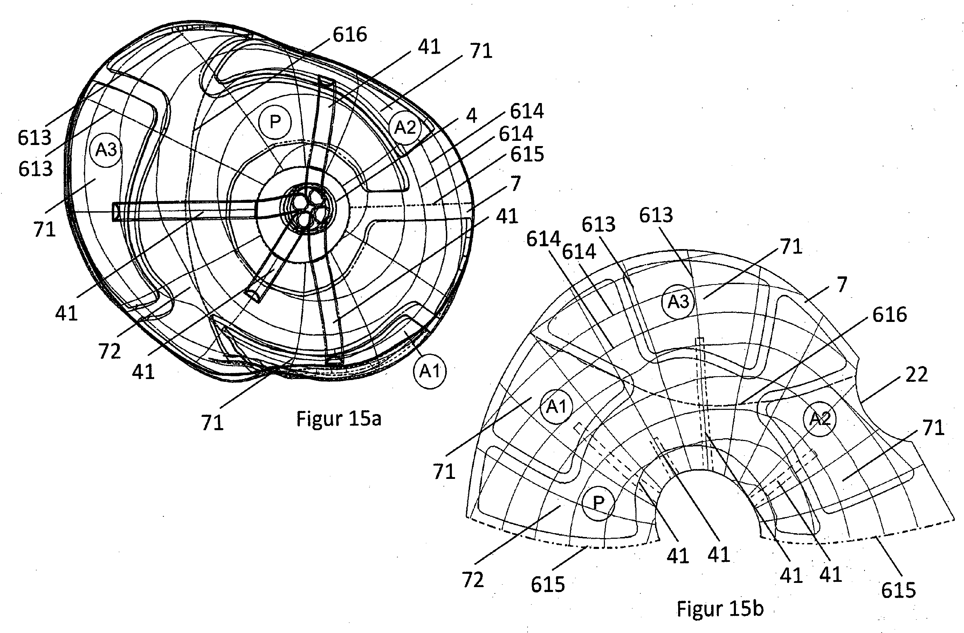

**Figur 15** zeigt eine Hülle (7) mit zumindest einer expandierbaren Einheit (71,72). Die 3D-Form der Hülle (7) in Figur 15a ist vergleichbar mit dem in Zusammenhang mit Figur 14a erläuterten 3D-Modell und zeigt ein wie oben beschriebenes Koordinatensystem mit Längengraden (613), Breitengraden (614), der Verlaufslinie des Septums (616) und der Schnittlinie (615) für die Darstellung der 2D-Abrollung. Die Hülle (7) kann ein Herz zumindest zum Teil umschließen. Die Hülle (7) kann zumindest zum Teil die Form eines Herzens haben. Die Hülle (7) kann eine ähnliche Form wie die Schale haben. Die Hülle kann in die Schale eingebracht werden. Die Hülle kann aus Kunststoff, Polymer, Kautschuk, Gummi, Latex, Silikon oder Polyurethan bestehen. Die Hülle kann maßgeschneidert auf Basis des in Figur 14a erläuterten 3D-Modells gefertigt werden. Geeignete Fertigungsverfahren sind beispielsweise Gießen oder insbesondere Thermoformen. Beim Thermoformen wird das in Platten oder Folien vorliegende Ausgangsmaterial der Hülle unter Wärmeeinwirkung an ein formgebendes physikalisches 3D-Modell des Herzens gepresst, gezogen und/oder gesaugt, wobei die Form des 3D-Modells auf das Material übertragen wird. Die Hülle (7) kann eine Dicke von 0,1 mm bis 1 mm, vorzugsweise 0,2 mm bis 0,5 mm aufweisen. Die Hülle weist eine Aussparung (22) auf, die in ihrer Form, Lage und Dimension im Wesentlichen mit der Aussparung in der Schale übereinstimmt, welche den anatomischen Bereich um die Vena cava inferior freistellt. Eine Kompromittierung der Vena cava inferior und eine folgliche Behinderung der Befüllung des rechten Vorhofs wird dadurch verhindert.

In Figur 15a ist die Hülle (7) mit zumindest einer expandierbaren Einheit (71, 72) als eine Hülle (7) mit einer Vielzahl von Kammern dargestellt. Figur 15b zeigt eine 2D-Abrollung des 3D-Modells aus Figur 15a. Die in Figur 15b dargestellte Abrollung entspricht im Wesentlichen der in Zusammenhang mit Figur 14b erläuterten Abrollung eines 3D-Modells. Anders als in Figur 14a ist das 3D-Modell in Figur 15a derart gedreht, dass eine Sicht von oben in die Hülle (7) ermöglicht wird. In den Figuren 15a und 15b sind beispielhaft vier expandierbare Einheiten (71, 72) dargestellt, von denen drei Augmentationseinheiten (71) sind und eine eine Positionierungseinheit (72) ist. Die expandierbaren Einheiten (71, 72) können strukturell ähnlich sein, können aber wie oben beschrieben verschiedenen Zwecken dienen.

Generell kann eine Augmentationseinheit (71) periodisch expandiert und entspannt werden damit ein Druck auf das Herz ausgeübt werden kann. Dieser Druck wird vorzugsweise in Bereichen auf den Herzmuskel ausgeübt, unter denen sich eine Herzkammer befindet. Durch Ausüben von Druck auf eine Herzkammer durch die Augmentationseinheit (71) wird die natürliche Pumpbewegung des Herzens unterstützt oder ersetzt, und das Blut im Herzen wird aus der Herzkammer in die abführende Arterie gepumpt. Ein von einer Augmentationseinheit (71) auf eine rechte Herzkammer ausgeübter Druck führt zum Auswurf des Blutes aus der rechten Herzkammer in die Lungenarterie. Ein von einer Augmentationseinheit (71) auf eine linke Herzkammer ausgeübter Druck führt zum Auswurf des Blutes aus der linken Herzkammer in die Aorta.

In Figur 15 sind drei Augmentationseinheiten (71) gezeigt, die sich in Bereichen am oberen Rand der Hülle (7) befinden. Jede der Augmentationseinheiten (71) wird in diesem Beispiel von einer eigenen Leitung (41) versorgt. Im Falle von Augmentationseinheiten (71) in Form von inflatierbaren Kammern sind die Leitungen (41) vorzugsweise pneumatische oder hydraulische Leitungen. Andere Ausführungsformen umfassen eine, zwei, drei, vier, fünf, sechs oder mehr Augmentationseinheiten (71), die mit einer, zwei, drei, vier, fünf, sechs oder mehr Leitungen (41) versorgt werden. Die zumindest eine Leitung (41) kann aus Kunststoff, Polymer, Kautschuk Gummi, Latex, Silikon oder Polyurethan bestehen. Die zumindest eine Leitung (41) kann über, neben oder unter zumindest einer Augmentationseinheit (71) verlaufen. Die zumindest eine Leitung (41) kann vorzugsweise unter einer Positionierungseinheit (72) verlaufen, damit sich keine Druckstellen zwischen der zumindest einen Leitung (41) und der Herzwand ergeben. Die zumindest eine Leitung (41) kann auch über oder neben einer Positionierungseinheit (72) verlaufen.

Die in Figur 15 dargestellten Augmentationseinheiten (71) A1, A2, und A3 befinden sich an einem Bereich am oberen Rand der Hülle (7) und werden jeweils von einer eigenen Leitung (41) versorgt. Die Augmentationseinheiten (71) A1 und A2 können wie in diesem Ausführungsbeispiel derart positioniert sein, dass sie eine linke Herzkammer unterstützen können. In dieser Darstellung ist die Augmentationseinheit A2 (71) in ihrer Form und Position so angepasst, dass sie den Bereich der Aussparung (22) nicht überdeckt. Die anatomische Lage der Vena cava inferior kann von Patient zu Patient geringfügig variieren, bzw. andere anatomische Gegebenheiten können es erforderlich machen, dass auch andere Augmentationseinheiten (71) oder eventuell vorhandene Positionierungseinheiten (72) an eine Aussparung (22) angepasst werden müssen. Die Augmentationseinheit (71) A3 ist derart positioniert, dass sie eine rechte Herzkammer unterstützten kann. Die einzelnen Augmentationseinheiten (71) A1, A2 und A3 können individuell expandiert werden. Die Augmentationseinheiten (71) A1 und A2 können zur Unterstützung einer Linksherzinsuffizienz dienen. Die Augmentationseinheit (71) A3 kann zur Unterstützung einer Rechtsherzinsuffizienz dienen. Die Augmentationseinheiten (71) A1, A2 und A3 können zur Unterstützung einer beidseitigen Herzinsuffizienz dienen. Die Augmentationseinheiten (71) können synchron oder asynchron expandiert werden. Vorzugsweise kann die Expansion der Augmentationseinheiten (71) aufeinander abgestimmt werden, dass eine natürliche Pumpfunktion des Herzens unterstützt wird.

Eine Positionierungseinheit (72) ist eine Einheit die ebenfalls expandiert werden kann. Vorzugsweise wird eine Positionierungseinheit während des Betriebs der Vorrichtung zur Unterstützung der Funktion eines Herzens mehr statisch als periodisch expandiert. Die Positionierungseinheit (72) kann expandiert werden, um die Vorrichtung am Herzen zu fixieren und die Passgenauigkeit der Vorrichtung zu optimieren. Mit Hilfe einer Positionierungseinheit (72) kann auch auf Veränderungen des Herzmuskels reagiert werden. Baut sich der Herzmuskel ab oder auf, kann eine Positionierungseinheit (72) weiter expandiert oder entspannt werden, um eine optimale Passform zu gewährleisten.

In Figur 15 ist eine Positionierungseinheit (72) dargestellt, die die Zwischenräume zwischen den drei Augmentationseinheiten (71) auf der Hülle (7) im Wesentlichen ausfüllt. Die Positionierungseinheit (72) kann von einer oder mehreren Augmentationseinheiten (71) einen seitlichen Abstand von 1 mm, 3 mm, 5 mm, 10 mm, 20 mm, 30 mm, 40 mm oder mehr haben. Die Positionierungseinheit kann aber auch unabhängig von den Augmentationseinheiten geformt sein. So kann die Positionierungseinheit beispielsweise auch nur den Bereich nahe der Herzspitze umschließen ohne die Zwischenräume zwischen den Augmentationseinheiten auszufüllen. Die Positionierungseinheit (72) kann mit einer eigenen Leitung (41), im Falle von einer mit Fluid befüllbaren Kammer mit einer eigenen pneumatischen oder hydraulischen Leitung, versorgt werden. Andere Ausführungsformen umfassen eine, zwei, drei, vier, fünf, sechs oder mehr Positionierungseinheiten (72), die mit einer, zwei, drei, vier, fünf, sechs oder mehr pneumatischen oder hydraulischen Leitungen (41) versorgt werden. Die Leitung (41) kann aus Kunststoff, Polymer, Kautschuk Gummi, Latex, Silikon oder Polyurethan bestehen. Die zumindest eine Leitung (41) zum Versorgen der Positionierungseinheit (72) kann unter der Positionierungseinheit (72) verlaufen. Die in Figur 15 dargestellte eine Positionierungseinheit (72) füllt Bereiche zwischen den Augmentationseinheiten (71) aus. Die dargestellte Positionierungseinheit (72) hat Fortsätze, die in die Zwischenräume zwischen den Augmentationseinheiten (71) ragen.

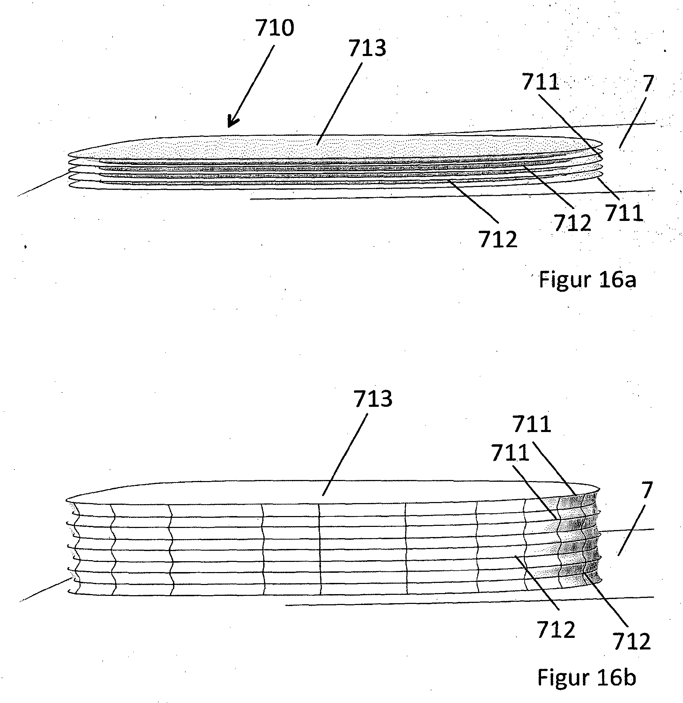

**Figur 16** zeigt eine expandierbare Einheit (71, 72) in Form einer Kammer (710). Die dargestellte Kammer ist eine balgförmige Kammer (710). Eine balgförmige Kammer (710) hat zumindest einen Abschnitt der in Form eines Balges ist. Vorzugsweise handelt es sich um einen Faltenbalg, der aus ein, zwei, drei, vier, fünf, sechs, sieben oder mehr Falten besteht. Als eine Falte kann eine nach außen gerichtete Knickkante (711) definiert werden. Als eine Falte kann eine nach innen gerichtete Knickkante (712) definiert werden. Eine, mehrere oder alle Knickkanten (711, 712) können verstärkt sein. Eine Verstärkung einer Knickkante (711, 712) ist vorteilhaft, da die Knickkante (711, 712) durch das Expandieren und Entspannen der Kammer (710) erhöhten Belastungen ausgesetzt sein kann. Eine Verstärkung einer oder mehrerer Knickkanten (711, 712) kann Materialermüdungen entlang der zumindest einen Knickkante (711, 712) reduzieren oder verhindern. Eine Verstärkung einer Knickkante (711, 712) kann durch eine höhere Wandstärke des Materials an der Knickkante (711, 712) erreicht werden. Eine Verstärkung einer Knickkante (711, 712) kann auch durch Aufbringen von zusätzlichem Material erfolgen, wobei das aufgebrachte Material das gleiche Material wie das darunterliegende Material sein kann, oder wobei das aufgebrachte Material ein anderes Material als das darunterliegende Material sein kann. Eine Kammer (710) kann eine Oberseite (713), eine Unterseite und eine Seitenfläche aufweisen, wobei die Seitenfläche bevorzugt balgförmig ausgebildet ist. Die Ober- (713) und/oder die Unterseite kann oval, kreisförmig, elliptisch oder polygonal sein. Die Oberseite (713) kann eine andere Form haben als die Unterseite.

Eine balgförmige Kammer (710) kann in eine wie oben beschriebene Schale eingebracht werden. Die Kammer (710) kann direkt in der Schale befestigt oder fixiert werden. Die Kammer (710) kann an strukturellen Elementen der Schale, wie zum Beispiel einem Draht eines Drahtgeflechts, einer Strebe eines Gitterwerks, oder einer Struktur auf einem Schalenmantel befestigt werden. Die Kammer (710) kann an Kreuzungspunkten eines Geflechts oder Gitterwerks befestigt werden. Die Schale kann wie oben beschrieben mit einer Membran bedeckt sein. In diesen Fällen kann die Kammer (710) auch an der Membran befestigt werden. Die Membran kann auch eine Unterseite der Kammer (710) sein.

Die balgförmige Kammer (710) kann auch auf einer Hülle (7) befestigt sein. Mehrere balgförmige Kammern (710) können auf einer Hülle (7) befestigt sein. Die Hülle (7) kann zumindest zum Teil die Form eines Herzens haben. Die Hülle (7) kann eine ähnliche Form wie die Schale haben. Die Hülle (7) kann in die Schale eingebracht werden. Die Hülle (7) kann in der Schale befestigt und/oder fixiert werden. Die Hülle (7) kann neben einer oder mehrerer Augmentationseinheiten, wie z.B. einer oder mehreren balgförmigen Kammern (710), auch eine oder mehrere Positionierungseinheiten aufweisen. Die Unterseite der Kammer (710) kann aus dem gleichen Material gefertigt sein wie die Hülle (7). Die Hülle (7) kann Teil der Kammer (710) sein. Die Hülle (7) kann die Unterseite der Kammer formen. In solchen Fällen werden auf eine Hülle (7) nur die Seitenflächen aufgebracht, die balgförmig sein können. Zusätzlich kann eine Oberseite (713) angebracht werden. Die Oberseite (713) kann auch eine Hülle sein. Ausführungsformen bestehen aus zwei Hüllen (7), wobei die Hüllen (7) die Oberseite und die Unterseite der Kammern bilden und Seitenflächen zwischen den Hüllen geformt werden. Seitenflächen können in diesem Fall auch durch Verbinden, insbesondere durch Verschweißen oder Verkleben, der beiden Hüllen geformt werden. Die Hüllen (7) können derart miteinander verbunden, insbesondere verschweißt oder verklebt werden, dass eine Kammer gebildet wird. Die zumindest eine Leitung, die die Kammer versorgt, kann ähnlich wie die Kammer zumindest zum Teil auch durch Verbinden der beiden Hüllen (7), insbesondere durch Verschweißen oder Verkleben der beiden Hüllen (7) geformt werden. Auf einer der beiden Hüllen (7) oder auf beiden Hüllen (7) können sich ein oder mehrere Sensoren oder eine oder mehrere Elektroden befinden.

Die Hülle (7) mit der zumindest einen expandierbaren Einheit kann am oberen Rand und/oder am unteren Rand zumindest eine Tasche aufweisen. Die zumindest eine Tasche kann über eine strukturelle Form einer Schale zumindest teilweise gestülpt werden. Die Tasche kann beispielsweise über eine Schlaufe eines Drahtgeflechts oder einen Bügel eines Gitterwerks zumindest teilweise gestülpt werden.

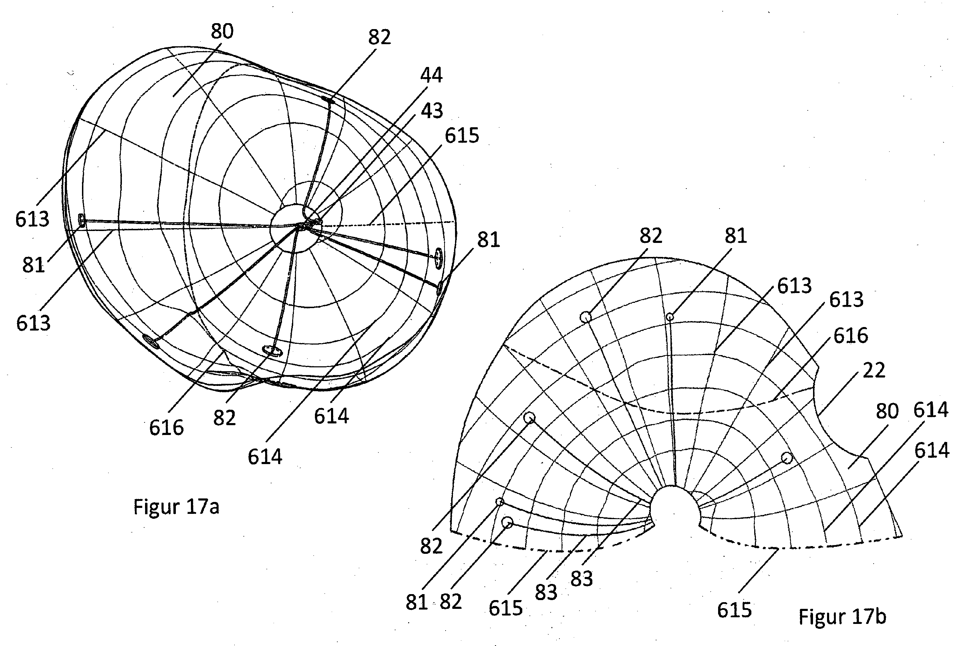

Die Hülle (7) mit der zumindest einen expandierbaren Einheit kann einen Wirkstoff enthalten. Beispielsweise kann die Hülle (7) einen antithrombotischen Wirkstoff, einen anti-proliferativen Wirkstoff, einen anti-inflammatorischen Wirkstoff, einen anti-neoplastischen Wirkstoff, einen anti-mitotischen Wirkstoff, einen anti-mikrobiellen Wirkstoff, einen Biofilm-Syntheseinhibitor, einen Antibiotika-Wirkstoff, einen Antikörper, einen antikoagulierenden Wirkstoff, einen cholesterol-senkenden Wirkstoff, einen beta-Blocker, oder eine Kombination davon enthalten. Vorzugsweise ist der Wirkstoff in Form einer Beschichtung auf der Hülle (7). Die Hülle (7) kann auch mit extrazellulären Matrix-Proteinen, insbesondere Fibronektin oder Kollagen, beschichtet sein. **Figur 17** zeigt eine Hülle (80) mit zumindest einem Sensor (81) und/oder zumindest einer Elektrode (82). Die 3D-Form der Hülle (80) in Figur 17a ist vergleichbar mit dem in Figur 14a erläuterten 3D-Modell und zeigt ein wie oben beschriebenes Koordinatensystem. Die Hülle (80) kann ein Herz zumindest zum Teil umschließen. Die Hülle (80) kann zumindest zum Teil die Form eines Herzens haben. Die Hülle (80) mit dem zumindest einen Sensor (81) und/oder zumindest einer Elektrode (82) kann eine ähnliche Form wie die Schale haben. Die Hülle (80) kann in die Schale eingebracht werden. Die Hülle (80) kann eine Aussparung (22) haben, die in ihrer Form, Lage und Dimension im Wesentlichen mit der Aussparung in der Schale übereinstimmt. Die Hülle (80) kann aus Kunststoff, Polymer, Kautschuk Gummi, Latex, Silikon oder Polyurethan bestehen. Die Hülle (80) kann eine Dicke von 0,1 mm bis 1 mm, vorzugsweise 0,2 mm bis 0,5 mm aufweisen. Die Hülle (80) kann beschichtet sein, insbesondere mit einem Gleitmittel, das die Reibung zwischen dem Herzmuskel und der Hülle (80) mit dem zumindest einen Sensor (81) und/oder der zumindest einer Elektrode (82) vermindert.

Die Hülle (80) mit dem zumindest einen Sensor (81) und/oder zumindest einer Elektrode (82) kann anstelle oder zusätzlich zur Hülle mit den expandierbaren Einheiten eingebracht werden. Falls die Hülle (80) zusätzlich zur Hülle mit den expandierbaren Einheiten eingebracht wird, so kann sich die Hülle (80) näher zum Herzen befinden als die Hülle mit den expandierbaren Einheiten. Die Hülle (80) mit dem zumindest einen Sensor (81) und/oder zumindest einer Elektrode (82) kann dabei fest zu den expandierbaren Einheiten verbunden sein, womit die Hülle (80) dann die Oberseite der expandierbaren Einheiten bildet. Die Hülle (80) mit dem zumindest einen Sensor (81) und/oder zumindest einer Elektrode (82) kann auch nur am oberen und/oder am unteren Rand mit der Hülle mit den expandierbaren Einheiten und/oder der Schale verbunden sein, womit die Hülle (80) durch die Hülle mit den expandierbaren Einheiten gegen den Herzmuskel gedrückt werden kann. Eine Beschichtung, insbesondere eine Beschichtung mit einem Gleitmittel, kann auch zwischen der Hülle (80) mit dem zumindest einen Sensor (81) und/oder der zumindest einer Elektrode (82) und der Hülle mit der zumindest einen expandierbaren Einheit vorhanden sein. Der zumindest eine Sensor (81) und/oder die zumindest eine Elektrode (82) kann in die Hülle (80) eingearbeitet, eingegossen oder eingeschweißt sein, oder auf der Hülle (80) angebracht, aufgeklebt oder aufgenäht sein. Der zumindest eine Sensor (81) und/oder die zumindest eine Elektrode (82) können mit Verstärkungen versehen sein, die ein Knicken während dem Komprimieren der Vorrichtung verhindern können.

In Figur 17a ist die Hülle (80) mit zumindest einem Sensor (81) und/oder zumindest einer Elektrode (82) als eine Hülle (80) mit einer Vielzahl von Sensoren (81) und Elektroden (82) dargestellt. Figur 17b zeigt eine 2D-Abrollung des 3D-Modells aus Figur 17a. Die in Figur 17b dargestellte Abrollung entspricht im Wesentlichen der in Zusammenhang mit Figur 14b erläuterten Abrollung eines 3D-Modells. Anders als in Figur 14a ist das 3D-Modell in Figur 17a derart gedreht, dass eine Sicht von oben in die Hülle (80) ermöglicht wird. In den Figuren 17a und 17b sind beispielhaft acht Sensoren (81) oder Elektroden (82) gezeigt. Andere Ausführungsformen können einen, zwei, drei, vier, fünf, sechs, sieben, acht, neun, zehn, elf, zwölf oder mehr Sensoren (81) und/oder Elektroden (82) umfassen. Die Hülle (80) mit zumindest einem Sensor (81) oder zumindest einer Elektrode (82) kann ein Netz von Sensoren (81) oder Elektroden (82) sein. Das Netz von Sensoren (81) oder Elektroden (82) kann das Herz zumindest zum Teil umschließen. Die Sensoren (81) oder Elektroden (82) in dem Netz von Sensoren (81) oder Elektroden (82) können miteinander verbunden sein. Die Hülle (80) kann als Träger für das Netz von Sensoren (81) oder Elektroden (82) fungieren. Das Netz von Sensoren (81) oder Elektroden (82) kann auch nur teilweise auf einer Hülle (80) angebracht sein. Das Netz von Sensoren (81) oder Elektroden (82) kann auch ohne eine Hülle (80) in eine wie oben beschriebene Schale eingebracht werden.

Der zumindest eine Sensor (81) oder die zumindest eine Elektrode (82) kann eine physikalische oder eine chemische Eigenschaft seiner Umgebung bestimmen. Die Eigenschaft kann qualitativ oder quantitativ erfasst werden. Der Sensor (81) kann ein aktiver Sensor oder ein passiver Sensor sein. Der zumindest eine Sensor (81) kann zumindest einen Parameter des Herzens erfassen. Der zumindest eine Sensor (81) kann geeignet sein, die Herzfrequenz, den Herzkammerdruck, den systolischen Blutdruck oder den diastolischen Blutdruck zu bestimmen. Der Sensor (81) kann geeignet sein, den von einer expandierbaren Einheit auf eine Fläche ausgeübten Druck, den pH-Wert, den elektrischen Widerstand, die Osmolarität einer Lösung, die Sauerstoffsättigung oder den Durchfluss durch ein Gefäß zu messen. Der zumindest eine Sensor kann auch als Elektrode verwendet werden.

Die zumindest eine Elektrode (82) kann geeignet sein Bereiche des Herzens zu stimulieren und/oder das Aktionspotential am Herzmuskel während des Erregungsprozesses zu messen. Insbesondere kann die zumindest eine Elektrode (82) geeignet sein den Herzmuskel mit Hilfe von elektrischen Impulsen zu stimulieren. Eine elektrische Stimulation kann einen Herzmuskel zu einer Kontraktion anregen. Die zumindest eine Elektrode (82) kann eine Herzschrittmacherelektrode sein. Die Elektrode (82) kann eine extrakardiale Stimulationselektrode sein. Der Herzmuskel kann mit einer Elektrode (82) vor, während oder nach einer Unterstützung der Pumpfunktion des Herzens durch eine Schale mit zumindest einer expandierbaren Einheit stimuliert werden. Die Expansion einer expandierbaren Einheit kann vor, während oder nach einer Stimulation mit einer Elektrode (82) ablaufen. Die Vorrichtung zur Unterstützung der Funktion eines Herzens kann nur mit zumindest einer expandierbaren Einheit oder nur durch Stimulation mit zumindest einer Elektrode (82) betrieben werden. Ein gleichzeitiger Betrieb der zumindest einen expandierbaren Einheit und der zumindest einen Elektrode (82) kann synchron oder asynchron sein. Die zumindest eine Elektrode kann auch als Sensor verwendet werden.

Der zumindest eine Sensor (81) oder die zumindest eine Elektrode (82) kann auf der Hülle (80) befestigt sein. Der zumindest eine Sensor (81) oder die zumindest eine Elektrode (82) kann auf die Hülle (80) geklebt, genäht oder geschweißt sein. Der zumindest eine Sensor (81) oder die zumindest eine Elektrode (82) kann in der Hülle (80) angebracht, vorzugsweise eingeschweißt sein. Der zumindest eine Sensor (81) oder die zumindest eine Elektrode (82) können dabei so positioniert werden, dass sie genau über einer Augmentationseinheit liegen oder genau nicht über einer Augmentationseinheit liegen. Der zumindest eine Sensor (81) oder die zumindest eine Elektrode (82) können auch so positioniert werden, dass sie genau über einer Positionierungseinheit liegen oder sich in einem Bereich befinden unter dem sich keine expandierbare Einheit befindet. Bei Nichtvorhandensein der Hülle (80) können zumindest ein Sensor (81) oder zumindest eine Elektrode (82) auch an entsprechenden Stellen der Hülle mit der zumindest einen expandierbaren Einheit und/oder auf den expandierbaren Einheiten angebracht werden.

Der zumindest eine Sensor (81) oder die zumindest eine Elektrode (82) kann über eine Leitung (84) mit einer Versorgungseinheit verbunden sein. Die vom Sensor (81) oder der Elektrode (82) ermittelten Daten können auch verbindungslos über eine Funktechnik wie beispielsweise bluetooth übermittelt werden. Die Kontakte der Elektroden oder Sensoren oder die gesamte Hülle können mit einer Substanz beschichtet werden, die die Leitfähigkeit erhöhen oder verbessern. Beispielsweise kann eine Graphitbeschichtung auf den Kontakten deren Leitfähigkeit erhöhen.

### Beispiel 1

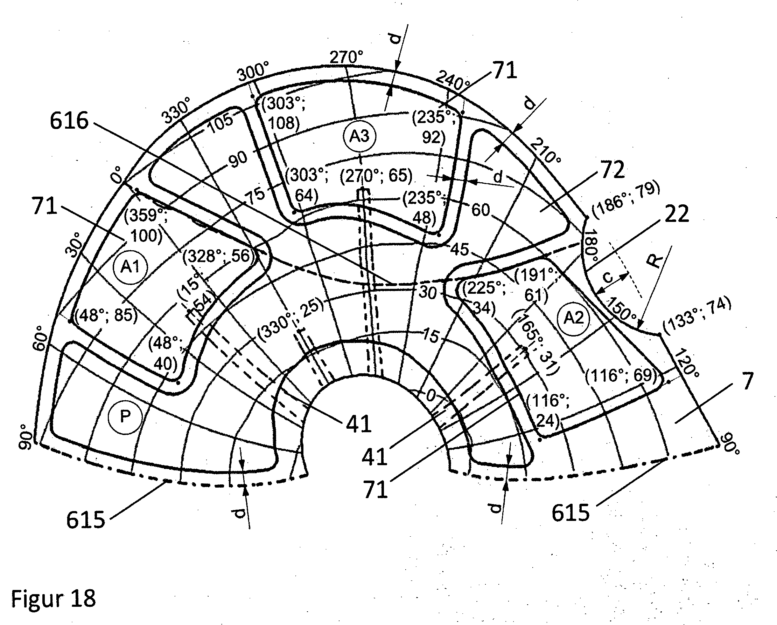

**Figur 18** zeigt ein Ausführungsbeispiel für eine Hülle (7) mit zumindest einer expandierbaren Einheit (71, 72). Dargestellt in Figur 18 ist eine wie in Zusammenhang mit Figur 14 beschriebene 2D Abrollung eines 3D-Modells. Die dargestellte Hülle dieses Ausführungsbeispiels umfasst drei Augmentationseinheiten (71) (A1, A2, A3) und eine Positionierungseinheit (72) (P). In diesem Ausführungsbeispiel nehmen die Augmentationseinheiten A1 und A2 jeweils eine Fläche von 28,6 cm² auf der Hülle ein. Die Fläche die von der Augmentationseinheit A3 eingenommen wird, beträgt in diesem Beispiel 34,5 cm². In diesem Ausführungsbeispiel nimmt die Positionierungseinheit (72) (P) eine Fläche von 114,5 cm² ein. Unter normalen Bedingungen beträgt die nominale Expansion der Positionierungseinheit (P) 5 mm, d.h. die Positionierungseinheit ist teilweise expandiert und weist eine Dicke von 5 mm auf. Die Positionierungseinheit kann eine Kammer sein, die mit einem Fluid befüllt und entfüllt werden kann. Die Dicke der Positionierungseinheit kann dadurch zwischen 1 mm und 10 mm betragen, vorzugsweise zwischen 3 mm und 7 mm. Durch Veränderung der Dicke der Positionierungseinheit (72) (P) kann eine Größenzunahme oder -abnahme des Herzens kompensiert werden und der korrekte Sitz der Hülle (7) und/oder Schale bleibt im Wesentlichen gewährleistet. Die Augmentationseinheiten A1 und A2 können in diesem Beispiel etwa 1,9 cm in ihren Dicken expandiert werden, um einen Druck auf eine Herzkammer, hier einer linken Herzkammer, aufzubauen. Die effektive Volumenexpansion der Augmentationseinheiten A1 und A2 ist in diesem Beispiel 40 ml. Die effektive Volumenexpansion der Augmentationseinheit A3 beträgt in diesem Fall 50 ml und führt zu einer effektiven Dickenexpansion von 1,45 cm. Jede der Ecken einer Augmentationseinheit kann durch die Koordinaten der Eckpunkte definiert werden. Das Koordinatensystem wurde in Zusammenhang mit Figur 14 erläutert.

Die Augmentationseinheit A1 verläuft im vorliegenden Beispiel von Eckpunkt 1 (ϕ=359°; z=100) über Eckpunkt 2 (ϕ=48°; z=85) und Eckpunkt 3 (ϕ=48°; z=40) zu Eckpunkt 4 (ϕ=328°; z=56) und liegt im implantierten Zustand am linken Ventrikel an. Die Verbindung des Eckpunktes 1 mit Eckpunkt 2 verläuft im Wesentlichen parallel zum oberen Rand der Hülle (7) mit einem Abstand (d) von etwa 5 mm. Die Verbindung des Eckpunkts 2 mit Eckpunkt 3 verläuft im Wesentlichen entlang der ϕ=48° Linie. Die Verbindung des Eckpunktes 3 mit Eckpunkt 4 verläuft im Wesentlichen parallel zu dem im 3D-Modell gezeigten oberen Rand der Hülle (7). Die Verbindung des Eckpunktes 4 mit dem Eckpunkt 1 verläuft im Wesentlichen entlang der Septumslinie (616). Die Ecken der Augmentationseinheit A1 sind abgerundet und beschreiben einen kreisförmigen Bogen mit einem Durchmesser von 4 mm.

Die Form und Lage der Augmentationseinheit A2 wurde in diesem Beispiel an die Aussparung (22) angepasst. Die Aussparung (22) verläuft dabei am äußeren Rand von Koordinate (ϕ=186°; z=79) zu (ϕ=133°; z=74). Die Form der Aussparung ist in diesem Beispiel so gewählt, dass die Aussparung im dreidimensionalen Raum eine runde Form mit dem Radius R=33 mm beschreibt. In der abgerollten Darstellung kann die Aussparung (22) daher etwas von der kreisrunden Form abweichen. Der maximale Abstand der Aussparung von der Linie des gedachten nicht-unterbrochenen Randes beträgt hier c=13 mm. Die Augmentationseinheit A2 verläuft im vorliegenden Beispiel von Eckpunkt 1 (ϕ=116°; z=69) über Eckpunkt 2 (ϕ=191°; z=61) und Eckpunkt 3 (ϕ=225°; z=34) zu Eckpunkt 4 (ϕ=116°; z=24) und liegt im implantierten Zustand am linken Ventrikel an. Die Verbindung des Eckpunktes 1 mit Eckpunkt 2 verläuft in diesem Beispiel nicht mehr parallel zum oberen Rand der Hülle (7). Die Verbindungsgerade der beiden Punkte verläuft von Eckpunkt 1 ausgehend schräg vom oberen Rand weg, so dass ein minimaler Abstand von 3mm zur Aussparung (22) eingehalten wird. Bei Eckpunkt 2 schneidet diese Verbindungslinie die Septumslinie (616). Die Verbindung des Eckpunktes 2 mit dem Eckpunkt 3 verläuft im Wesentlichen entlang der Septumslinie (616). Die Verbindung des Eckpunktes 3 mit Eckpunkt 4 verläuft im Wesentlichen parallel zu dem im 3D-Modell gezeigten oberen Rand der Hülle (7). Die Verbindung des Eckpunktes 4 mit dem Eckpunkt 1 verläuft im Wesentlichen entlang der ϕ=116° Linie. Die Ecken der Augmentationseinheit A2 sind abgerundet und beschreiben einen kreisförmigen Bogen mit einem Durchmesser von 4 mm.

Die Augmentationseinheit A3 verläuft im vorliegenden Beispiel von Eckpunkt 1 (ϕ=235°; z=92) über Eckpunkt 2 (ϕ=303°; z=108) und Eckpunkt 3 (ϕ=303°; z=64) zu Eckpunkt 4 (ϕ=235°; z=48) und liegt im implantierten Zustand am rechten Ventrikel an. Die Verbindung des Eckpunktes 1 mit Eckpunkt 2 verläuft im Wesentlichen parallel zum oberen Rand der Hülle (7) mit einem Abstand (d) von etwa 5 mm. Die Verbindung des Eckpunktes 2 mit dem Eckpunkt 3 verläuft im Wesentlichen entlang der ϕ=303° Linie. Die Verbindung des Eckpunktes 3 mit Eckpunkt 4 verläuft im Wesentlichen parallel zu dem im 3D-Modell gezeigten oberen Rand der Hülle (7). Die Verbindung des Eckpunktes 4 mit dem Eckpunkt 1 verläuft im Wesentlichen entlang der ϕ=235° Linie. Die Ecken der Augmentationseinheit A3 sind abgerundet und beschreiben einen kreisförmigen Bogen mit einem Durchmesser von 4 mm.

Die Positionierungseinheit P im Ausführungsbeispiel der Figur 18 ist derart gestaltet, dass sie die Zwischenräume zwischen den Augmentationseinheiten (71) auf der Hülle (7) im Wesentlichen ausfüllt. Die Positionierungseinheit (72) kann auch beschrieben werden als eine Positionierungseinheit (72) mit Fortsätzen, die die von den Augmentationseinheiten nicht ausgefüllten Bereiche auf der Hülle (7) im Wesentlichen ausfüllt. Im Ausführungsbeispiel hat die Positionierungseinheit P im Wesentlichen einen seitlichen Abstand (d) von den Augmentationseinheiten (71) und dem oberen Rand der Hülle (7) von etwa 5 mm. Ebenfalls ist die Positionierungseinheit (72) von der Schnittlinie (615) beabstandet, was vorteilhaft bei der Herstellung sein kann. Wird die Hülle (7) mit der zumindest einen expandierbaren Einheit in einem zweidimensionalen Zustand geformt, können sämtliche Augmentationseinheiten (71) und Positionierungseinheiten (72) auf der Hülle (7) angebracht werden, bevor die Hülle (7) in eine dreidimensionale Form gerollt wird.

Die Leitungen (41), die die expandierbaren Einheiten (71,72) versorgen, sind im Ausführungsbeispiel von Figur 18 hydraulische oder pneumatische Leitungen (41), die radial vom unteren Rand der Schale zu den Augmentationseinheiten verlaufen. Die Leitung (41) für die Augmentationseinheit A1 verläuft entlang der Linie ϕ=15° und endet in einer Höhe von z=54. Die Leitung (41) für die Augmentationseinheit A2 verläuft entlang der Linie ϕ=165° und endet in einer Höhe von z=31. Die Leitung (41) für die Augmentationseinheit A3 verläuft entlang der Linie ϕ=270° und endet in einer Höhe von z=65. Die Leitung (41) für die Positionierungseinheit P verläuft entlang der Linie ϕ=330° und endet in einer Höhe von z=25.

### Beispiel 2

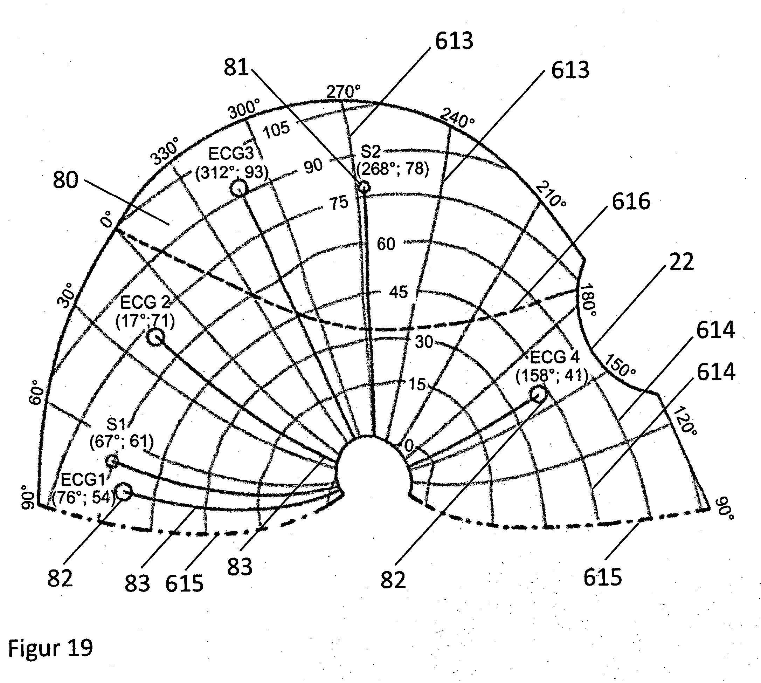

**Figur 19** zeigt ein Ausführungsbeispiel einer Positionierung von zumindest einem Sensor (81) und/oder einer Elektrode (82). Dargestellt in Figur 19 ist eine wie in Zusammenhang mit Figur 14 beschriebene Abrollung eines 3D-Modells. Die dargestellte Hülle (80) dieses Ausführungsbeispiels umfasst sechs Sensoren (81) oder Elektroden (82), wobei zwei davon Sensoren (Sensor S1, S2) (81) und vier Elektrokardiogramm-Elektroden (EKG, electrocardiogramm electrode ECG1, ECG2, ECG3, ECG4) (82) sind. In diesem Ausführungsbeispiel befinden sich der zumindest eine Sensor (81) und/oder die zumindest eine Elektrode auf einer Hülle (80) ohne expandierbare Einheiten. Der zumindest eine Sensor (81) und/oder die zumindest eine Elektrode (82) können auch analog der folgenden Beschreibung direkt an den entsprechenden Positionen der Hülle mit der zumindest einen expandierbaren Einheit angebracht werden. Die Hülle (80) kann aus Kunststoff, Polymer, Kautschuk Gummi, Latex, Silikon oder Polyurethan bestehen. Die Hülle (80) kann eine Dicke von 0,1 mm bis 1 mm, vorzugsweise 0,2 mm bis 0,5 mm aufweisen. Die vier Drucksensoren (81) können in die Hülle (80) eingearbeitet sein, insbesondere eingegossen oder eingeschweißt sein. Die Sensoren (81) können mit Verstärkungen versehen sein, die ein Knicken während dem Komprimieren der Vorrichtung verhindern können. Die EKG-Elektroden (82) können auf der dem Herzen zugewandten Seite der Hülle (80) angebracht sein. In dem Ausführungsbeispiel in Figur 19 ist ein Koordinatensystem wie in Zusammenhang mit Figur 14 beschrieben dargestellt. Mit Hilfe des Koordinatensystems können die Positionen der Sensoren (81) und Elektroden (82) für dieses Ausführungsbeispiel wie folgt bestimmt werden: der Sensor S1 befindet sich an der Koordinate (ϕ=67°; z=61), der Sensor S2 befindet sich an der Koordinate (ϕ=268°; z=78). Die EKG-Elektrode ECG1 befindet sich an der Koordinate (ϕ=76°; z=54), die EKG-Elektrode ECG2 befindet sich an der Koordinate (ϕ=17°; z=71), die EKG-Elektrode ECG3 befindet sich an der Koordinate (ϕ=312°; z=93) und die EKG-Elektrode ECG4 befindet sich an der Koordinate (ϕ=158°; z=48). Für kleinere bzw. größere Herzen bleiben die Winkelkoordinaten für die Sensoren (81) und/oder Elektroden (82) im Wesentlichen gleich, der z-Wert ist für kleinere bzw. größere Herzen um einen Faktor zu skalieren. Für kleinere Herzen kann ein Skalierungsfaktor zwischen 0,85 und 0,95 liegen und für große Herzen kann ein Skalierungsfaktor zwischen 1,05 und 1,15 liegen. In diesem Beispiel liegen der Sensor S2 und die Elektroden ECG2 und ECG4 über einer Augmentationseinheit. Der Sensor S1 und die Elektroden ECG1 und ECG3 befinden sich nicht über Augmentationseinheiten. Der Bereich der Aussparung (22) wird nicht von Sensoren, Elektroden oder deren Zuleitungen überdeckt.

### Bevorzugte Ausführungsformen

Bevorzugte Ausführungsformen werden in den folgenden Absätzen definiert.
1. Vorrichtung zur Unterstützung der Funktion eines Herzens, umfassend:
   eine Schale,
   eine Hülle mit zumindest einer expandierbaren Einheit.
2. Vorrichtung nach Ausführungsform 1, wobei die expandierbare Einheit einen Druck auf eine Fläche ausüben kann.
3. Vorrichtung nach einer der vorherigen Ausführungsformen, wobei die expandierbare Einheit eine Kammer ist.
4. Vorrichtung nach Ausführungsform 3, wobei die Kammer eine Augmentationseinheit oder eine Positionierungseinheit ist.
5. Vorrichtung nach Ausführungsform 1, wobei zumindest ein Sensor auf der Hülle angebracht ist, der geeignet ist, die Herzfrequenz, die Sauerstoffsättigung, den Herzkammerdruck, den systolischen Blutdruck, den diastolischen Blutdruck, oder eine Kombination davon zu messen.
6. Vorrichtung nach Ausführungsform 5, wobei der Sensor geeignet ist, den Druck in einer Augmentationseinheit, den Druck in einer Positionierungskammer, den von einer expandierbaren Kammer gegen eine Fläche ausgeübten Druck, das Vorhandensein von Flüssigkeit, den pH-Wert, den elektrischen Widerstand, die Osmolarität einer Lösung oder den Durchfluss durch ein Gefäß zu messen.
7. Vorrichtung nach einer der Ausführungsformen 5 oder 6, wobei der Sensor eine EKG-Elektrode, ein Drucksensor, ein pH-Sensor, ein Flüssigkeitssensor oder ein Durchflusssensor ist.
8. Vorrichtung nach einer der vorhergehenden Ausführungsformen, wobei die Vorrichtung eine zweite Hülle umfasst, die einen oder mehrere Sensoren gemäß den Ausführungsformen 5 bis 7 umfasst.
9. Vorrichtung nach einer der vorhergehenden Ausführungsformen, wobei die Hülle und die Schale eine im Wesentlichen gleiche Form haben.
10. Vorrichtung nach einer der vorhergehenden Ausführungsformen, wobei die Hülle und die Schale derart gestaltet sind, dass deren oberer Rand im implantierten Zustand etwa 1 mm bis 3 cm, insbesondere etwa 3 mm bis 2 cm, insbesondere 5 mm bis 10 mm unterhalb der Herzklappenebene liegt.
11. Vorrichtung zur Unterstützung der Funktion eines Herzens nach einer der vorhergehenden Ausführungsformen, wobei die Wandstärke der Hülle 0,1 mm bis 1 mm, insbesondere 0,2 mm bis 0,5 mm beträgt.
12. Vorrichtung nach einer der vorhergehenden Ausführungsformen, wobei die Form der Hülle der Form des durch einen Faktor räumlich gestreckten 3D-Abbildes des Herzens entspricht, insbesondere, wobei der Streckungsfaktor in einem Bereich von 1,004 bis 1,1 oder in einem Bereich von 1,005 bis 1,06 ist.
13. Vorrichtung nach einer der vorhergehenden Ausführungsformen, wobei die Hülle oder die Schale einen pharmazeutischen Wirkstoff umfasst, vorzugsweise wobei der Wirkstoff einen antithrombotischen Wirkstoff, einen anti-proliferativer Wirkstoff, einen anti-inflammatorischer Wirkstoff, einen anti-neoplastischer Wirkstoff, einen anti-mitotischer Wirkstoff, einen anti-mikrobieller Wirkstoff, einen Biofilm-Syntheseinhibitor, einen Antibiotika-Wirkstoff, einen Antikörper, einen antikoagulierender Wirkstoff, einen cholesterolsenkenden Wirkstoff, einen beta-Blocker, oder eine Kombination davon umfasst.
14. Vorrichtung nach einer der vorhergehenden Ausführungsformen, wobei die Hülle mit einem Gleitmittel zur Verminderung von Reibung beschichtet ist, insbesondere wobei die Beschichtung eine Beschichtung mit Graphit oder eine reibungsvermindernde Silikonbeschichtung sein.
15. Vorrichtung nach einer der vorhergehenden Ausführungsformen, wobei der zumindest eine Sensor und/oder die zumindest eine Elektrode oder deren Leitungen mit Verstärkungen versehen sind, die ein Knicken verhindern können.
16. Vorrichtung nach einer der vorhergehenden Ausführungsformen, wobei die zumindest eine expandierbare Einheit auf der Oberseite oder auf der Unterseite der Hülle angebracht ist.
17. Vorrichtung nach einer der vorhergehenden Ausführungsformen, wobei die Schale mit einer Membran bedeckt ist.
18. Vorrichtung nach einer der vorhergehenden Ausführungsformen, wobei die Schale Befestigungsvorrichtungen für eine Hülle hat.
19. Vorrichtung nach einer der vorhergehenden Ausführungsformen, wobei die Schale aus einem Drahtgeflecht oder Gitterwerk besteht.
20. Vorrichtung nach Ausführungsform 19, wobei das Drahtgeflecht oder das Gitterwerk an der oberen Seite der Schale zumindest eine Schlaufe hat.
21. Vorrichtung nach Ausführungsform 20, wobei die Hülle an der oberen Seite zumindest eine Lasche hat, die in die zumindest eine Schlaufe der Schale eingehängt werden kann.
22. Vorrichtung nach Ausführungsform 1, wobei die Hülle an ihrem oberen Rand üm die Schale gestülpt wird.
23. Vorrichtung nach Ausführungsform 22, wobei der um die Schale gestülpte Teil der Hülle zum Fixieren verklebt oder verschweißt werden.
24. Vorrichtung nach einer der vorhergehenden Ausführungsformen 19 bis 23, wobei das Drahtgeflecht oder das Gitterwerk Kreuzungspunkte hat, an denen zumindest eine der Hülle fixiert werden kann.
25. Vorrichtung nach einer der vorhergehenden Ausführungsformen, wobei die Schale an der unteren Spitze eine Öffnung hat und die Hülle an der unteren Spitze durch die Öffnung durchragt.
26. Vorrichtung nach einer der vorhergehenden Ausführungsformen 25, wobei 3 mm bis 2 cm der Hülle durch die Öffnung durchragt.
27. Vorrichtung nach einer der vorhergehenden Ausführungsformen wobei die Hülle an der unteren Spitze der Schale fixiert werden kann.
28. Vorrichtung nach einer der vorhergehenden Ausführungsformen, wobei die Hülle zumindest teilweise aus einem Kunststoff, insbesondere Polyurethan, Silikon, Polytetrafluorethylen oder einer Polyethylen- oder Polypropylen-Folie, geformt ist.
29. Vorrichtung nach einer der vorhergehenden Ausführungsformen, wobei die Hülle einen pharmazeutischen Wirkstoff beinhaltet, insbesondere wobei die herzzugewandte Hülle mit dem pharmazeutischen Wirkstoff beschichtet ist.
30. Vorrichtung nach einer der vorhergehenden Ausführungsformen, wobei die Hülle zumindest eine Elektrode umfasst.
31. Vorrichtung nach einer der vorhergehenden Ausführungsformen, wobei die Hülle patienten-spezifisch hergestellt wird, insbesondere mittels Thermoformen.
32. Vorrichtung nach Ausführungsform 1, wobei die zumindest eine expandierbare Einheit sich im oberen Bereich der Hülle nahe der Herzklappenebene befindet.
33. Vorrichtung nach Ausführungsform 1, wobei die zumindest eine expandierbare Einheit eine aktiv expandierbare Einheit ist, insbesondere wobei die zumindest eine expandierbare Einheit durch Befüllen mit einem Fluid expandiert werden kann.
34. Vorrichtung nach Ausführungsform 1, wobei die Vorrichtung weiter eine Steuereinheit umfasst.
35. Vorrichtung nach Ausführungsform 34, wobei die Vorrichtung einen EKG Sensor umfasst und die Steuereinheit die Daten des EKG Sensors verwendet um die zumindest eine expandierbare Einheit gezielt anzusteuern.
36. Vorrichtung nach Ausführungsform 1, wobei die Schale und die Hülle minimal-invasiv implantiert werden kann.
37. Vorrichtung nach Ausführungsform 1, wobei die Schale weiter zumindest eine Verlängerungsstrebe umfasst.
38. Vorrichtung nach Ausführungsform 37, wobei die zumindest eine Verlängerungsstrebe Teil der Schale ist, insbesondere die Verlängerung eines Drahtes des Geflechtes oder die Verlängerung einer Strebe des Gitterwerkes, oder, wobei die zumindest eine Verlängerungsstrebe an die Schale gekoppelt werden kann.
39. Vorrichtung nach Ausführungsform 37 oder 38, wobei die Schale zwei, drei, vier, fünf, sechs oder mehr Verlängerungsstreben umfasst.
40. Vorrichtung nach Ausführungsform 37 bis 39, wobei die zumindest eine Verlängerungsstrebe zwischen 1 cm und 10 cm, insbesondere zwischen 4 cm und 7 cm lang ist.
41. Vorrichtung nach einer der vorhergehenden Ausführungsformen, wobei die Schale an ihrem oberen Rand eine Aussparung aufweist, die derart geformt ist, dass die Schale im implantierten Zustand die Vena cava nicht berührt.
42. Vorrichtung nach einer der vorhergehenden Ausführungsformen, wobei die Aussparung eine Länge entlang des oberen Schalenrandes von 2 cm, 3 cm, 4 cm, 5 cm, 6 cm, 7 cm, oder mehr hat.
43. Vorrichtung nach einer der vorhergehenden Ausführungsformen, wobei die Aussparung eine Tiefe zwischen 1mm und 40mm hat, insbesondere zwischen 3mm und 15mm hat.
44. Vorrichtung nach einer der Ausführungsform 41 bis 43 wobei die Aussparung bogenförmig, halbkreisförmig, rechteckig oder polygonal ist.
45. Vorrichtung nach Ausführungsform 41, wobei die Hülle eine Aussparung umfasst, die mit der Aussparung der Schale in Deckung gebracht werden kann.
46. Vorrichtung nach Ausführungsform 1, wobei die Vorrichtung nur eine Hülle und keine weitere Hülle umfasst.
47. Vorrichtung nach einer der vorhergehenden Ausführungsformen, weiter umfassend eine Versorgungseinheit.
48. Vorrichtung nach Ausführungsform 47, weiter umfassend ein Kabel zum Verbinden der zumindest einen expandierbaren Einheit oder des zumindest einen Sensors mit der Versorgungseinheit.
49. Vorrichtung nach Ausführungsform 48, weiter umfassend einen Stecker zum Verbinden der Vorrichtung zur Unterstützung der Funktion eines Herzens mit der Versorgungseinheit.
50. Vorrichtung zur Unterstützung der Funktion eines Herzens nach einer der vorhergehenden Ausführungsformen, weiter umfassend einen Verschluss zum Abdichten eines Membrandurchbruches, insbesondere zum Abdichten einer Öffnung im Perikard.
51. Vorrichtung zur Unterstützung der Funktion eines Herzens nach einer der vorhergehenden Ausführungsformen, weiter umfassend ein Zuführsystem.
52. Verfahren zum Herstellen einer Vorrichtung zur Unterstützung der Funktion eines Herzens, umfassend:
   Bereitstellen eines virtuellen oder reellen Abbildes eines Herzens,
   Formen einer Schale in der Form des Abbildes des Herzens,
   Formen einer Hülle in der Form des Abbildes des Herzens,
   Einbringen der Hülle in die Schale.
53. Zuführsystem zum Zuführen der Vorrichtung gemäß einer der vorhergehenden Ausführungsformen.

## Patentansprüche

1. Vorrichtung zur Unterstützung der Funktion eines Herzens, umfassend:
eine Schale (2),
eine Hülle (7) mit zumindest einer expandierbaren Einheit (71, 72),
**dadurch gekennzeichnet, dass** die Schale selbst-expandierend ist und aus einem Gitterwerk besteht, das zumindest zum Teil aus einer Formgedächtnislegierung gefertigt ist.

2. Vorrichtung nach Anspruch 1, wobei die expandierbare Einheit (71, 72) einen Druck auf eine Fläche ausüben kann.

3. Vorrichtung nach Anspruch 1, wobei zumindest ein Sensor (81) auf der Hülle oder der expandierbaren Einheit angebracht ist, der geeignet ist, die Herzfrequenz, die Sauerstoffsättigung, den Herzkammerdruck, den systolischen Blutdruck, den diastolischen Blutdruck, oder eine Kombination davon zu messen.

4. Vorrichtung nach Anspruch 1, wobei die Vorrichtung eine zweite Hülle umfasst, die einen oder mehrere Sensoren gemäß Anspruch 3 umfasst.

5. Vorrichtung nach einem der vorhergehenden Ansprüche, wobei die Schale an der oberen Seite der Schale zumindest eine Schlaufe (26, 28) hat.

6. Vorrichtung nach Anspruch 1, wobei die Hülle (7) an ihrem oberen Rand um die Schale (2) gestülpt werden kann.

7. Vorrichtung nach einem der vorhergehenden Ansprüche, wobei die Schale (2) an der unteren Spitze eine Öffnung hat und die Hülle (7) an der unteren Spitze durch die Öffnung durchragt.

8. Vorrichtung nach einem der vorhergehenden Ansprüche, wobei die Hülle (7) patientenspezifisch hergestellt wird, insbesondere mittels Thermoformen.

9. Vorrichtung nach Anspruch 1, wobei die zumindest eine expandierbare Einheit (71, 72) sich im oberen Bereich der Hülle (7) nahe der Herzklappenebene befindet und wobei die zumindest eine expandierbare Einheit (71, 72) eine aktiv expandierbare Einheit ist.

10. Vorrichtung nach Anspruch 1, wobei die Vorrichtung weiter eine Steuereinheit und einen EKG Sensor (81) umfasst und die Steuereinheit die Daten des EKG Sensors verwendet um die zumindest eine expandierbare Einheit gezielt anzusteuern.

11. Vorrichtung nach Anspruch 1, wobei die Schale (2) weiter zumindest eine Verlängerungsstrebe (23) umfasst.

12. Vorrichtung nach einem der vorhergehenden Ansprüche, wobei die Schale an ihrem oberen Rand eine Aussparung (22) aufweist, die derart geformt ist, dass die Schale im implantierten Zustand die Vena cava inferior nicht berührt.

13. Vorrichtung nach Anspruch 12, wobei die Hülle (7) ebenfalls eine Aussparung aufweist deren Form, Größe und Position im Wesentlichen mit der Aussparung in der Schale übereinstimmt.

14. Verfahren zum Herstellen einer Vorrichtung zur Unterstützung der Funktion eines Herzens, umfassend:
Bereitstellen eines virtuellen oder reellen Abbildes eines Herzens,
Formen einer Schale (2) in der Form des Abbildes des Herzens, wobei die Schale selbst-expandierend ist und aus einem Gitterwerk besteht, das zumindest zum Teil aus einer Formgedächtnislegierung gefertigt ist,
Formen einer Hülle (7) in der Form des Abbildes des Herzens,
Einbringen der Hülle (7) in die Schale (2).

## Claims

1. A cardiac assistance device comprising
a shell (2),
a sleeve (7) with at least one expandable unit (71, 72),
**characterised in that**
the shell is self-expanding and consists of a latticework, which is at least partially made of a shape-memory alloy.

2. Device according to claim 1, wherein the expandable unit (71, 72) can apply pressure to a surface.

3. Device according to claim 1, wherein at least one sensor (81) is located on the sleeve or on the expandable unit, with the sensor being suitable to measure heart frequency, oxygen saturation, pressure in the ventricle, systolic blood pressure, diastolic blood pressure or a combination thereof.

4. Device according to claim 1, wherein the device includes a second sleeve comprising one or more sensors according to claim 3.

5. Device according to one of the preceding claims, wherein the shell has at the upper edge of the shell at least one loop (26, 28).

6. Device according to claim 1, wherein the sleeve (7) at its upper edge can be wrapped around the shell (2).

7. Device according to one of the preceding claims, wherein the shell (2) has an opening at its lower tip and the sleeve (7) protrudes through the opening at the lower tip.

8. Device according to one of the preceding claims, wherein the sleeve (7) is manufactured patient-specifically, in particular by thermoforming.

9. Device according to claim 1, wherein the at least one expandable unit (71, 72) is positioned at the upper portion of the sleeve (7) close to the heart valve plane and wherein the at least one expandable unit (71, 72) is an active expandable unit.

10. Device according to claim 1, wherein the device further comprises a control unit and an ECG-sensor (81), the control unit processing the data from the ECG-sensor in order to control the at least one expandable unit.

11. Device according to claim 1, wherein shell (2) further comprises at least one extension strut (23).

12. Device according to one of the preceding claims, wherein the shell features a cut-out (22) at its upper edge, which is shaped such that the shell in its implanted state does not touch the vena cava inferior.

13. Device according to claim 12, wherein the sleeve (7) also features a cut-out which substantially corresponds to shape, dimension and position of the cut-out in the shell.

14. Method of manufacturing a cardiac assistance device comprising:
providing a virtual or real image of a heart,
forming of a shell (2) in the shape of the image of a heart, whereas the shell is self-expanding and consists of a latticework, which is at least partially made of a shape-memory alloy,
forming a sleeve (7) in the shape of the image of a heart,
inserting of the sleeve (7) into the shell (2).

## Revendications

1. Ensemble d'assistance au fonctionnement du coeur, comprenant :
une coque (2),
une enveloppe (7) dotée d'au moins une unité expansible (71, 72),
**caractérisé en ce que**
la coque est auto-expansible et est constituée d'un treillis réalisé au moins en partie à partir d'un alliage à mémoire de forme.

2. Ensemble selon la revendication 1, dans lequel l'unité expansible (71, 72) peut exercer une pression sur une surface.

3. Ensemble selon la revendication 1, dans lequel au moins un capteur (81) est placé sur l'enveloppe ou sur l'unité expansible et permet de mesurer la fréquence cardiaque, la saturation en oxygène, la pression dans la cavité cardiaque, la pression sanguine systolique, la pression sanguine diastolique ou une de leurs combinaisons.

4. Ensemble selon la revendication 1, dans lequel l'ensemble comporte une deuxième enveloppe qui comporte un ou plusieurs capteurs selon la revendication 3.

5. Ensemble selon l'une des revendications précédentes, dans lequel la coque présente au moins une boucle (26, 28) sur le côté supérieur de la coque.

6. Ensemble selon la revendication 1, dans lequel l'enveloppe (7) peut être rabattue autour de la coque (2) sur son bord supérieur.

7. Ensemble selon l'une des revendications précédentes, dans lequel la coque (2) présente une ouverture à sa pointe inférieure et en ce que l'enveloppe (7) traverse l'ouverture sur la pointe inférieure.

8. Ensemble selon l'une des revendications précédentes, dans lequel l'enveloppe (7) est réalisée spécifiquement pour un patient, en particulier par thermoformage.

9. Ensemble selon la revendication 1, dans lequel au moins une unité expansible (71, 72) est située à proximité du plan des valves cardiaques dans la partie supérieure de l'enveloppe (7) et dans lequel la ou les unités expansibles (71, 72) sont des unités activement expansibles.

10. Ensemble selon la revendication 1, dans lequel l'ensemble présente en outre une unité de commande et un capteur ECG (81) et en ce que l'unité de commande utilise les données du capteur ECG pour commander de manière contrôlée la ou les unités expansibles.

11. Ensemble selon la revendication 1, dans lequel la coque (2) comporte en outre au moins un passant de prolongement (23).

12. Ensemble selon l'une des revendications précédentes, dans lequel la coque présente sur son bord supérieur une découpe (22) configurée de telle sorte qu'à l'état implanté, la coque ne soit pas en contact avec la veine cave inférieure.

13. Ensemble selon la revendication 12, dans lequel l'enveloppe (7) présente également une découpe dont la forme, la taille et la position correspondent essentiellement à celles de la découpe ménagée dans la coque.

14. Procédé de fabrication d'un dispositif d'assistance au fonctionnement du coeur, comprenant les étapes qui consistent à :
préparer une copie virtuelle ou réelle d'un coeur,
façonner une coque (2) à la forme de la copie du coeur, la coque étant auto-expansible et étant formée d'un treillis réalisé au moins en partie en un alliage à mémoire de forme,
façonner une enveloppe (7) sous la forme de la copie du coeur et
placer l'enveloppe (7) dans la coque (2).
